# EUROPEAN PATENT APPLICATION

(11) **EP 4 083 227 A1**
(43) Date of publication of application: **02.11.2022**
(21) Application number: 21382377.6
(22) Date of filing: 29.04.2021
(51) Int. Cl.: C12Q 1/6853, C12Q 1/6865

(54) **LINEAR DNA WITH ENHANCED RESISTANCE AGAINST EXONUCLEASES**

(71) Applicant: 4basebio, S.L.U., 28049 Cantoblanco (Madrid) (ES); 4basebio Discovery Limited, Over Cambridge CB24 5QE (GB)
(72) Inventor: LANCKRIET, Heikki, UNITED KINGDOM, CB24 5QE (GB); PICHER, Angel, 28049 Cantoblanco (Madrid) (ES)
(74) Representative: Boult Wade Tennant LLP

(57) **Abstract**

The present invention relates to linear double-stranded DNA products comprising nuclease-resistant nucleotides at internal positions in each strand. In addition, the present invention relates to complex molecules, nanoparticles, compositions and libraries comprising the linear double-stranded DNA products. Also provided are methods for producing and using the linear double-stranded DNA products.

## Description

### TECHNICAL FIELD

The present invention relates to linear double-stranded DNA products comprising nuclease-resistant nucleotides at internal positions in each strand. In addition, the present invention relates to complex molecules, nanoparticles, compositions and libraries comprising the linear double-stranded DNA products. Also provided are methods for producing and using the linear double-stranded DNA products.

### BACKGROUND

Native nucleic acids are susceptible to enzymatic degradation in a biological environment. Exonucleases cleave mononucleotides from the end of a polynucleotide chain by hydrolysing phosphodiester bonds at either the 3'- or the 5'-end of the polynucleotide chain. For example, exonuclease III removes mononucleotides from the 3'-end of the polynucleotide chain, while exonuclease VIII cleaves the polynucleotide chain in a 5' to 3' direction. Meanwhile, the phosphodiester bonds within a polynucleotide chain are cleaved by the action of endonucleases.

Considerable efforts have been made to extend the effective molecular lifetime of nucleic acids by generating nucleic acid analogues capable of resisting digestion by both extracellular and intracellular nucleases. One of the proposed solutions includes the use of phosphorothioated nucleotides (i.e. 2'-deoxynucleotides-5'-(α-thio)-triphosphate).

Phosphorothioated nucleotides comprise a sulphur atom instead of a non-bridging oxygen atom. These modified nucleotides show comparable physical and chemical characteristics to corresponding unmodified nucleotides, but are resistant to exonuclease digestion. As such, the incorporation of the phosphorothioate functional group can prolong the half-life of the nucleic acid molecule.

Phosphorothioate modifications are used in nucleic acid drug development programmes. In therapeutic nucleic acids, the phosphorothioated nucleotides are incorporated into short, single-stranded polynucleotide chains. For example, an antisense oligonucleotide fomivirsen is a 21-mer phosphorothioate oligodeoxynucleotide used to treat cytomegalovirus retinitis (Stein and Castanotto, "FDA-approved oligonucleotide therapies in 2017." Molecular Therapy 25.5 (2017): 1069-1075). Similarly, pegaptanib (brand name Macugen) is a short (27-nucleotides) aptamer with a phosphorothioate 3'-3' deoxythymidine cap used for treating age-related macular degeneration of the retina.

Phosphorothioate modifications have also been used in the context of a linear double-stranded polynucleotide chain (e.g. double-stranded DNA) to cap the ends of the polynucleotide chain to increase resistance to exonuclease digestion (Putney et al. "A DNA fragment with an alpha-phosphorothioate nucleotide at one end is asymmetrically blocked from digestion by exonuclease III and can be replicated in vivo." Proceedings of the National Academy of Sciences 78.12 (1981): 7350-7354). To cap the ends of the polynucleotide chain, the ends are digested with a restriction enzyme and treated with a DNA polymerase and a mixture of deoxyribonucleotide triphosphates (dNTPs), at least one type of which is a phosphorothioated nucleotide complementary to a nucleotide in the overhanging strand. Since DNA polymerases add nucleotides in the 5' to 3' direction, the result of this treatment is a blunt-ended polynucleotide fragment with a phosphorothioated nucleotide located at the 3'-end of each strand (i.e. in "the cap").

US Patent US10350307 B2 describes the incorporation of phosphorothioated nucleotides into a circular double-stranded concatemeric DNA which comprises a plurality of tandem repeat sequences. The concatemeric DNA product of US Patent US10350307 B2 provides multiple copies of the expression sequences in a single circular product to yield an improved system for protein expression. The size and polydisperse nature of the concatemeric DNA product of US Patent US10350307 B2 makes it less suitable for use in nanoparticle production and/or assembly of viral or non-viral vectors.

Thus, a need exists for a linear double-stranded DNA product with enhanced resistance to nuclease digestion (e.g. exonuclease digestion) which is suitable for RNA production and protein expression. In addition, there remains a need for a linear double-stranded DNA product with enhanced resistance to nuclease digestion which is suitable for nanoparticle production, and/or assembly of viral or non-viral vectors and library generation.

### DESCRIPTION

The invention provides a linear double-stranded DNA product with enhanced resistance to nuclease digestion. The invention is based on the presence of nuclease-resistant nucleotides (i.e. protected nucleotides) in the DNA product. Preferably, the linear double-stranded DNA product comprises one or more nucleotides that are resistant to exonuclease (e.g. exonuclease III) digestion. More preferably still, the linear double-stranded DNA product comprises nucleotides that are resistant to exonuclease (e.g. exonuclease III) digestion at internal positions in one strand or in each strand of the DNA product. The linear double-stranded DNA product may comprise a cassette. The cassette may comprise a coding sequence. The location of protected nucleotides at internal positions in one strand or in each strand may be outside of the cassette. This location of protected nucleotides ensures that the cassette remains intact upon exonuclease digestion (e.g. exonuclease III digestion). The exonuclease-resistant nucleotides (i.e. protected nucleotides) may be phosphorothioated nucleotides.

The present inventors have surprisingly discovered a method for production of a linear double-stranded DNA product of enhanced resistance to nuclease digestion. Specifically, the linear double-stranded DNA product has enhanced resistance to exonuclease digestion (e.g. exonuclease III digestion). The enhanced resistance to exonuclease digestion extends the life of the linear double-stranded DNA product in a cell (i.e. the linear double-stranded DNA product has enhanced resistance to intracellular exonucleases) and in a cell-free system (i.e. the linear double-stranded DNA product has enhanced resistance to extracellular exonucleases). In addition, the present inventors have discovered a method for efficient introduction of protected nucleotides at both the 3'-end and the 5'-end of each of the DNA strand, which provides protection from digestion by exonucleases that cleave the 3'-end nucleotides (e.g. exonuclease III) and exonucleases that cleave the 5'-end nucleotides (e.g. exonuclease VIII). Thus, the linear double-stranded DNA product of the invention has prolonged in vivo expression when compared to a linear double-stranded DNA product that does not contain protected nucleotides.

The linear double-stranded DNA product of the present invention has additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes the linear double-stranded DNA product of the present invention particularly suitable for use in a pharmaceutical composition.

Unexpectedly, the present inventors have discovered a method for production of a linear double-stranded DNA product of enhanced resistance to nuclease digestion which allows for efficient production of large quantities of the linear double-stranded DNA product with enhanced resistance to exonuclease digestion. The large-scale manufacture of the product may be in a cell-free system, which results in a production of a pure sample comprising the linear double-stranded DNA product free of bacterial contaminants (e.g. remaining after cell lysis).

Importantly, the linear double-stranded DNA product of the present invention may be used to produce an improved cell delivery system, such as a viral vector or a nanoparticle. This is because the inventors of the present application have discovered a way to produce a linear double-stranded DNA product, which has low dispersity and which comprises a single copy of the gene of interest. Use of a linear double-stranded DNA product of low dispersity that comprises one copy of the gene of interest (or a collection of genes of interest) facilitates production of uniform cell delivery systems (i.e. cell delivery systems that are less disperse), such as nanoparticles, which is highly beneficial in view of improved cellular uptake and higher transfection efficiency.

### 1. Linear double-stranded DNA products

The invention provides a linear double-stranded DNA product comprising a sense strand and an antisense strand, wherein the linear double-stranded DNA product comprises one or more protected nucleotides (e.g. phosphorothioated nucleotides) at internal positions in each strand.

The internal positions may be any positions in the linear double-stranded DNA product other than the last nucleotide on the 3'-end and the 5'-end of the sense and antisense strands. The internal positions may be located at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA product. The internal positions may be located at least 7 nucleotides away from the 3'-end and/or the 5'-end of each strand of the linear double-stranded DNA product. Preferably, the internal positions are located at least 10 nucleotides away from the 3'-end and/or the 5'-end of each strand of the linear double-stranded DNA product. In the linear double-stranded DNA product, the internal positions may be located at least 7 nucleotides away from the 3'-end and the 5'-end of the sense strand. Preferably, in the linear double-stranded DNA product, the internal positions are located at least 10 nucleotides away from the 3'-end and the 5'-end of the sense strand. In the linear double-stranded DNA product, the internal positions may be located at least 7 nucleotides away from the 3'-end and the 5'-end of the antisense strand. Preferably, in the linear double-stranded DNA product, the internal positions may be located at least 10 nucleotides away from the 3'-end and the 5'-end of the antisense strand. In the linear double-stranded DNA product, the internal positions may be located at least 7 nucleotides away from the 3'-end of each strand. Preferably, in the linear double-stranded DNA product, the internal positions may be located at least 10 nucleotides away from the 3'-end of each strand. In the linear double-stranded DNA product, the internal positions may be located at least 7 nucleotides away from the 5'-end of each strand. Preferably, in the linear double-stranded DNA product, the internal positions may be located at least 10 nucleotides away from the 5'-end of each strand.

The linear double-stranded DNA product may not comprise a protected (e.g. phosphorothioated) nucleotide at a location that is close to the end of each strand of the DNA product. The protected (e.g. phosphorothioated) nucleotide may not be the 1^{st}, 2^{nd}, 3^{rd}, 4^{th}, 5^{th}, 6^{th}, 7^{th}, 8^{th}, 9^{th}, and/or 10^{th} nucleotide of the sense and/or the antisense strand from the 3'-end and/or the 5'-end. The linear double-stranded DNA product may not comprise a protected (e.g. phosphorothioated) nucleotide at either one of the positions 1-3, 1-4, 1-5, 1-6, 1-7, 1-8, 1-9, 1-10, 1-12, 1-14, 1-16, 1-18, or 1-20 of the sense and/or the antisense strand from the 3'-end and/or the 5'-end. Preferably, the linear double-stranded DNA product does not comprise a protected (e.g. phosphorothioated) nucleotide at positions 1-9 of the sense and/or the antisense strand from the 3'-end and/or the 5'-end. The linear double-stranded DNA product may not comprise a protected (e.g. phosphorothioated) nucleotide at positions 1-9 of the sense strand from the 3'-end and/or the 5'-end. The linear double-stranded DNA product may not comprise a protected (e.g. phosphorothioated) nucleotide at positions 1-9 of the antisense strand from the 3'-end and/or the 5'-end. For example, the first protected (e.g. phosphorothioated) nucleotide in the sense strand may be the 6^{th} nucleotide counting from the 5'-end of the sense strand. For example, the first protected (e.g. phosphorothioated) nucleotide in the antisense strand may be the 10^{th} nucleotide counting from the 3'-end of the antisense strand. The location of the first protected (e.g. phosphorothioated) nucleotide in each strand may be different. For example, the linear double-stranded DNA product may comprise the first protected (e.g. phosphorothioated) nucleotide at the 8^{th} position counting from the 3'-end of the sense strand and at the 12^{th} position counting from the 3'-end of the antisense strand. Similarly, the linear double-stranded DNA product may comprise protected (e.g. phosphorothioated) nucleotides which are located at the 8^{th} position counting from the 3'-end of the sense strand, the 6^{th} position counting from the 5'-end of the sense strand, the 12th position counting from the 3'-end of the antisense strand and the 7^{th} position counting from the 5'-end of the antisense strand. The location of the first protected (e.g. phosphorothioated) nucleotide in each strand may be the same. For example, the linear double-stranded DNA product may comprise the first protected (e.g. phosphorothioated) nucleotide at the 8^{th} position counting from the 3'-end of the sense strand and the 8^{th} position counting from the 3'-end of the antisense strand. The linear double-stranded DNA product may comprise a first protected (e.g. phosphorothioated) nucleotide which is the 6^{th}, 8^{th} or 10^{th} nucleotide counting from the 3'-end and/or the 5'-end of the sense and/or the antisense strand. The linear double-stranded DNA product may not comprise a first protected (e.g. phosphorothioated) nucleotide at positions 1-6 counting from the 3'-end and/or the 5'-end of the sense and/or the antisense strand. Thus, the internal positions may be any position within the linear double-stranded DNA product other than the last 6, 8 or 10 nucleotides at the 3'-end and the 5'-end of the sense and antisense strands. The linear double-stranded DNA product may comprise a first protected (e.g. phosphorothioated) nucleotide which is the 7^{th} nucleotide counting from the 3'-end and/or the 5'-end of the sense and/or the antisense strand. Preferably, the linear double-stranded DNA product may comprise a first protected (e.g. phosphorothioated) nucleotide which is the 10^{th} nucleotide counting from the 3'-end and/or the 5'-end of the sense and/or the antisense strand.

The linear double-stranded DNA product described herein may comprise a cassette. Thus, the invention also provides a linear double-stranded DNA product comprising a sense strand and an antisense strand, wherein the linear double-stranded DNA product comprises a single cassette and one or more protected (e.g. phosphorothioated) nucleotides at internal positions in each strand.

The term "single cassette" as used herein in the context of a linear double-stranded DNA product is intended to encompass a linear double-stranded DNA product that does not comprise or consist of a plurality of cassettes. That is to say that the linear double-stranded DNA product comprises only a single cassette, which may comprise a single coding sequence of a gene of interest. The single cassette may not comprise or consist of a plurality of tandem repeat sequences, and/or concatemeric DNA. For example, the linear double-stranded DNA product described herein may not comprise or consist of the concatemeric DNA which is produced by known methods, for example as described in US Patent US10350307 B2. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The cassette may comprise a coding sequence. The coding sequence may encode a gene of interest, for example a gene encoding a protein. For protein expression, the cassette comprising a coding sequence may be ligated into a vector or a plasmid comprising at least a portion of a promoter so that the portion of the promoter is operably linked to the coding sequence (e.g. the promoter may be upstream of the coding sequence). The vector or plasmid may further comprise a guide RNA (gRNA) of the Clustered Regularly Interspaced Short Palindromic Repeats (CRISPR) system. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The location of the protected (e.g. phosphorothioated) nucleotides in the linear double-stranded DNA product may be such that the cassette is protected from nuclease (e.g. exonuclease III) digestion. Thus, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The term *"5'-end nucleotide of the cassette"* as used herein is intended to encompass the 5'-end nucleotide of each strand of the cassette. Thus, in the linear double-stranded DNA product, the cassette typically comprises a 5'-end nucleotide of the sense strand and a 5'-end nucleotide of the antisense strand.

The term *"3'-end nucleotide of the cassette"* as used herein is intended to encompass the 3'-end nucleotide of each strand of the cassette. Thus, in the linear double-stranded DNA product, the cassette typically comprises a 3'-end nucleotide of the sense strand and a 3'-end nucleotide of the antisense strand.

The term *"outside of the cassette"* as used herein is intended to encompass any nucleotides which are not part of the cassette. This includes any nucleotides that are comprised in the linear double-stranded DNA product and which also do not form part of the cassette. The term *"N nucleotides outside of the cassette"* or *"N nucleotides away from the cassette"* is intended to describe nucleotides that are located N nucleotides from the end of the cassette towards the end of the linear double-stranded DNA product. For example, the term *"2 nucleotides outside of the cassette"* in the context of internal positions of nucleotides is meant to describe a nucleotide that is outside of the cassette and that is two nucleotides away from the last nucleotide of the cassette. For example, in the sequence: 5'-AAAAAACATAAAA (SEQ ID NO: 1), wherein the cassette starts from nucleotide "T" (in the 5' to 3' direction), the term *"2 nucleotides outside of the cassette"* refers to the "C" nucleotide. Thus, the term *"at least 2 nucleotides outside of the cassette"* or *"at least 2 nucleotides away from the cassette"* is meant to describe nucleotides that are outside of the cassette and that are at least two nucleotides away from the last nucleotide of the cassette. In the example above, nucleotides *"at least 2 nucleotides away from the cassette"* would be any nucleotides selected from 5'-AAAAAAC. Similarly, the term *"at least 2 nucleotides away from the 5'-end of the cassette"* is meant to describe nucleotides that are outside of the cassette and that are at least two nucleotides away from the last nucleotide at the 5'-end of the cassette. In the example above, the last nucleotide at the 5'-end of the cassette is "T", and nucleotides *"at least 2 nucleotides away from the 5'-end of the cassette"* would be any nucleotides selected from 5'-AAAAAAC.

The internal positions may not be located between the second and penultimate nucleotide of the cassette. The internal positions may be any position in the linear double-stranded DNA product other than the last nucleotide on the 3'-end and the 5'-end of the sense and antisense strands and other than nucleotides located between the second and penultimate nucleotide of the cassette. The internal positions may be located outside of the cassette and at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA product and/or at least 2, 3, 4, 5, 6, 7, 8, 9, 10, 12, 14, 16, 18, 20, 30, 40, 50, 75, or 100 nucleotides away (i.e. outside of the cassette) from the 3'-end and/or the 5'-end of each strand of the cassette. Preferably, the internal positions is located outside of the cassette and at least 6, at least 8, or at least 10 nucleotides away from the 3'-end and/or the 5'-end of each strand of the linear double-stranded DNA product and/or at least 6, at least 8, or at least 10 nucleotides away from the 3'-end and/or the 5'-end of each strand of the cassette (i.e. at least 6, at least 8, or at least 10 nucleotides outside of the cassette). Preferably, the cassette does not comprise a phosphorothioated nucleotide at either one of the positions 1-2, 1-3, 1-4, 1-5, 1-6, 1-7, 1-8, 1-9, 1-10, 1-12, 1-14, 1-16, 1-18, or 1-20 of the sense and/or the antisense strand away from the 3'-end and/or the 5'-end of the cassette (i.e. outside of the cassette). Preferably, the internal positions in each strand are located at least 6 nucleotides away from the end of the linear double-stranded DNA product and are not located between the second and penultimate nucleotide of the cassette. Preferably, the internal positions in each strand are located at least 10 nucleotides away from the end of the linear double-stranded DNA product and are not located between the second and penultimate nucleotide of the cassette. Preferably, the internal positions in each strand are located at least 6 nucleotides away from the end of the linear double-stranded DNA product and at least 6 nucleotides away from the end of the cassette (i.e. at least 6 nucleotides outside of the cassette). Preferably, the internal positions in each strand are located at least 10 nucleotides away from the end of the linear double-stranded DNA product and at least 10 nucleotides away from the end of the cassette (i.e. at least 10 nucleotides outside of the cassette). The linear double-stranded DNA product may comprise a first phosphorothioated nucleotide at an internal position which is a position other than the last nucleotide on the 3'-end and the 5'-end of the sense and antisense strands as long as this position is located at least 2, 3, 4, 5, 6, 7, 8, 9, 10, 12, 14, 16, 18, 20, 30, 40, or 50 nucleotides outside of the cassette. For example, the linear double-stranded DNA product may comprise a first phosphorothioated nucleotide which is at least the 6th, 8th or 10th nucleotide counting from the 3'-end and/or the 5'-end of the sense and/or the antisense strand as long as these positions are not located between the second and penultimate nucleotide of the cassette. Preferably, the linear double-stranded DNA product may comprise a first phosphorothioated nucleotide which is at least the 6th, 8th or 10th nucleotide counting from the 3'-end and/or the 5'-end of the sense and/or the antisense strand as long as these positions are located outside of the cassette. The linear double-stranded DNA product may comprise a first phosphorothioated nucleotide which is at least the 6th, 8th or 10th nucleotide counting from the 3'-end and/or the 5'-end of the sense and/or the antisense strand as long as these positions are located at least 2, 3, 4, 5, 6, 7, 8, 9, 10, 12, 14, 16, 18, 20, 30, 40, or 50 nucleotides outside of the cassette. Preferably, the linear double-stranded DNA product may comprise a first phosphorothioated nucleotide which is at least the 6th, 8th or 10th nucleotide counting from the 3'-end and/or the 5'-end of the sense and/or the antisense strand as long as these positions are located at least 6, 8, or 10 nucleotides outside of the cassette.

The linear double-stranded DNA product may comprise at least 2, 3, 4, 5, 6, 7, 8, 9 or 10 protected (e.g. phosphorothioated) nucleotides at internal positions in each strand. Preferably, the linear double-stranded DNA product comprises at least 2 protected (e.g. phosphorothioated) nucleotides at internal positions in each strand.

The invention provides a linear double-stranded DNA product comprising a sense strand and an antisense strand, wherein the linear double-stranded DNA product comprises a single cassette and at least two nuclease-resistant nucleotides (i.e. protected nucleotides) at internal positions in each strand, wherein the at least two nuclease-resistant nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The invention provides a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The linear double-stranded DNA product may be resistant to nuclease digestion or may have improved or enhanced resistance to nuclease digestion. The linear double-stranded DNA product may be resistant to exonuclease digestion or have improved or enhanced resistance to exonuclease digestion. The linear double-stranded DNA product may be resistant, or have improved or enhanced resistance, to digestion by exonucleases that cleave the 3'-end nucleotides (e.g. exonuclease III) and/or exonucleases that cleave the 5'-end nucleotides (e.g. exonuclease VIII). The terms "improved" or "enhanced" in the context of resistance to enzyme digestion refer to higher resistance to enzyme digestion when compared to a DNA product not produced by the methods described herein. For example, when compared to a product that does not comprise protected nucleotides, such as phosphorothioated nucleotides.

The location of phosphorothioated nucleotides in the sense strand of the linear double-stranded DNA product may be such that the one or more phosphorothioated nucleotides are located upstream of the cassette (i.e. towards the 5'-end of the sense strand of the DNA product).

In the sense strand of the linear double-stranded DNA product:
(a) one of the at least two phosphorothioated nucleotides may be the 5'-end nucleotide of the cassette; and/or
(b) one of the at least two phosphorothioated nucleotides may be in a first region of the sense strand, wherein the first region of the sense strand is 5' of the cassette.

The location of phosphorothioated nucleotides in the sense strand of the linear double-stranded DNA product may be such that the one or more phosphorothioated nucleotides are located downstream of the cassette (i.e. towards the 3'-end of the sense strand of the DNA product).

In the sense strand of the linear double-stranded DNA product:
(a) one of the at least two phosphorothioated nucleotides may be the 3'-end nucleotide of the cassette; and/or
(b) one of the at least two phosphorothioated nucleotides may be in a second region of the sense strand, wherein the second region of the sense strand is 3' of the cassette.

The location of phosphorothioated nucleotides in the antisense strand of the linear double-stranded DNA product may be such that the one or more phosphorothioated nucleotides are located upstream of the cassette (i.e. towards the 5'-end of the antisense strand of the DNA product).

In the antisense strand of the linear double-stranded DNA product:
(a) one of the at least two phosphorothioated nucleotides may be the 5'-end nucleotide of the cassette; and/or
(b) one of the at least two phosphorothioated nucleotides may be in a first region of the antisense strand, wherein the first region of the antisense strand is 5' of the cassette.

The location of phosphorothioated nucleotides in the antisense strand of the linear double-stranded DNA product may be such that the one or more phosphorothioated nucleotides are located downstream of the cassette (i.e. towards the 3'-end of the antisense strand of the DNA product).

In the antisense strand of the linear double-stranded DNA product:
(a) one of the at least two phosphorothioated nucleotides may be the 3'-end nucleotide of the cassette; and/or
(b) one of the at least two phosphorothioated nucleotides may be in a second region of the antisense strand, wherein the second region of the antisense strand is 3' of the cassette.

The location of phosphorothioated nucleotides in both the sense and the antisense strand of the linear double-stranded DNA product may be such that at least one phosphorothioated nucleotide is located downstream of the cassette and at least one phosphorothioated nucleotide is located upstream of the cassette in each strand.
In the linear double-stranded DNA product:
(a) in the sense strand one of the at least two phosphorothioated nucleotides may be the 5'-end nucleotide of the cassette, and/or one of the at least two phosphorothioated nucleotides may be in a first region of the sense strand, wherein the first region of the sense strand is 5' of the cassette;
(b) in the sense strand one of the at least two phosphorothioated nucleotides may be the 3'-end nucleotide of the cassette, and/or one of the at least two phosphorothioated nucleotides may be in a second region of the sense strand, wherein the second region of the sense strand is 3' of the cassette;
(c) in the antisense strand one of the at least two phosphorothioated nucleotides may be the 5'-end nucleotide of the cassette, and/or one of the at least two phosphorothioated nucleotides may be in a first region of the antisense strand, wherein the first region of the antisense strand is 5' of the cassette; and
(d) in the antisense strand one of the at least two phosphorothioated nucleotides may be the 3'-end nucleotide of the cassette, and/or one of the at least two phosphorothioated nucleotides may be in a second region of the antisense strand, wherein the second region of the antisense strand is 3' of the cassette.

The term *"first region of the sense strand'* as used herein is intended to encompass the part of the sense strand of the linear double-stranded DNA product that is between the 5'-end of the linear double-stranded DNA product and the first 5' nucleotide of the cassette in the sense strand. For example, in the sequence of the sense strand: 5'-AAAAAACATAAAA-3' (SEQ ID NO: 1), wherein the cassette starts from nucleotide "T" in the 5'-3' direction, the term *"first region of the sense strand'* refers to the 5'-AAAAAACA-3' region.

The term *"first region of the antisense strand'* is intended to encompass the part of the antisense strand of the linear double-stranded DNA product that is between the 5'-end of the linear double-stranded DNA product and the first 5' nucleotide of the cassette in the antisense strand. For example, in the sequence of the antisense strand: 5'-AAAAAACATAAAA-3' (SEQ ID NO: 1), wherein the cassette starts from nucleotide "T" in the 5'-3' direction, the term *"first region of the antisense strand'* refers to the 5'-AAAAAACA-3' region.

The term *"second region of the sense strand'* is intended to encompass the part of the sense strand of the linear double-stranded DNA product that is between the 3'-end of the linear double-stranded DNA product and the first 3' nucleotide of the cassette in the sense strand. For example, in the sequence of the sense strand: 5'-AAAAAACATAAAA-3' (SEQ ID NO: 1), wherein the cassette starts from nucleotide "T" in the 3'-5' direction, the term *"second region of the sense strand'* refers to the 5'-AAAA-3' region.

The term *"second region of the antisense strand'* is intended to encompass the part of the antisense strand of the linear double-stranded DNA product that is between the 3'-end of the linear double-stranded DNA product and the first 3' nucleotide of the cassette in the antisense strand. For example, in the sequence of the antisense strand: 5'-AAAAAACATAAAA-3' (SEQ ID NO: 1), wherein the cassette starts from nucleotide "T" in the 3'-5' direction, the term *"second region of the antisense strand'* refers to the 5'-AAAA-3' region.

The linear double-stranded DNA product may comprise a plurality of the phosphorothioated nucleotides upstream (i.e. towards the 5'-end of the sense strand of the DNA product) of the cassette. For example, the linear double-stranded DNA product may comprise at least 2, 3, 4, 5, 6, 7, 8, 9 or 10 phosphorothioated nucleotides upstream of the cassette. Preferably, at least 2 phosphorothioated nucleotides upstream of the cassette. Thus, the location of phosphorothioated nucleotides in the sense strand of the linear double-stranded DNA product may be such that at least two phosphorothioated nucleotides are located upstream of the cassette.

In the sense strand of the linear double-stranded DNA product:
(a) one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
(b) the at least two phosphorothioated nucleotides may be in a first region of the sense strand, wherein the first region of the sense strand is 5' of the cassette.

The linear double-stranded DNA product may comprise a plurality of the phosphorothioated nucleotides downstream (i.e. towards the 3-end of the DNA product) of the cassette. For example, the linear double-stranded DNA product may comprise at least 2, 3, 4, 5, 6, 7, 8, 9 or 10 phosphorothioated nucleotides downstream of the cassette, preferably at least 2 phosphorothioated nucleotides downstream of the cassette. Thus, the location of phosphorothioated nucleotides in the sense strand of the linear double-stranded DNA product may be such that at least two phosphorothioated nucleotides are located downstream of the cassette.

In the sense strand of the linear double-stranded DNA product:
(a) one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
(b) the at least two phosphorothioated nucleotides may be in a first region of the sense strand, wherein the first region of the sense strand is 3' of the cassette.

The location of phosphorothioated nucleotides in the antisense strand of the linear double-stranded DNA product may be such that at least two phosphorothioated nucleotides are located upstream of the cassette (i.e. towards the 5'-end of the antisense strand of the DNA product).

In the antisense strand of the linear double-stranded DNA product:
(a) one of the at least two phosphorothioated nucleotides is the 5'-end nucleotide of the cassette and one of the at least two phosphorothioated nucleotides is in a first region of the antisense strand, wherein the first region of the antisense strand is 5' of the cassette; or
(b) the at least two phosphorothioated nucleotides are in a first region of the antisense strand, wherein the first region of the antisense strand is 5' of the cassette.

The location of phosphorothioated nucleotides in the antisense strand of the linear double-stranded DNA product may be such that at least two phosphorothioated nucleotides are located downstream of the cassette (i.e. towards the 3'-end of the antisense strand of the DNA product).

In the antisense strand of the linear double-stranded DNA product:
(a) one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
(b) the least two phosphorothioated nucleotides may be in a second region of the antisense strand, wherein the second region of the antisense strand is 3' of the cassette.

Each strand of the linear double-stranded DNA product may comprise a plurality of phosphorothioated nucleotides. For example, the linear double-stranded DNA product may comprise at least 2, 3, 4, 5, 6, 7, 8, 9 or 10 phosphorothioated nucleotides upstream and downstream of the cassette. The location of phosphorothioated nucleotides in both the sense and the antisense strand of the linear double-stranded DNA product may be such that the at least two phosphorothioated nucleotides are located downstream of the cassette and at least two phosphorothioated nucleotides are located upstream of the cassette in each strand.

In the linear double-stranded DNA product:
(a) in the sense strand:
   i. one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
   ii. the at least two phosphorothioated nucleotides may be in a first region of the sense strand, wherein the first region of the sense strand is 5' of the cassette;
(b) in the sense strand:
   i. one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
   ii. the at least two phosphorothioated nucleotides may be in a second region of the sense strand, wherein the second region of the sense strand is 3' of the cassette;
(c) in the antisense strand:
   i. one of the at least two phosphorothioated nucleotides may be the 5'-end nucleotide of the cassette and one of the at least two phosphorothioated nucleotides may be in a first region of the antisense strand, wherein the first region of the antisense strand is 5' of the cassette; or
   ii. the at least two phosphorothioated nucleotides may be in a first region of the antisense strand, wherein the first region of the antisense strand is 5' of the cassette; and
(d) in the antisense strand:
   i. one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
   ii. the at least two phosphorothioated nucleotides may be in a second region of the antisense strand, wherein the second region of the antisense strand is 3' of the cassette.

The linear double-stranded DNA product may be resistant to nuclease digestion or may have improved or enhanced resistance to nuclease digestion. The linear double-stranded DNA product may be resistant to 3'-end exonuclease digestion (e.g. by exonuclease III) and/or 5'-end exonuclease digestion (e.g. by exonuclease VIII). The linear double-stranded DNA product may comprise a protected nucleotide (e.g. phosphorothioated nucleotide) at the 5'-end or at the 5'-end region. Preferably, the linear double-stranded DNA product comprises, optionally in each strand, a phosphorothioated nucleotide at the 5'-end or at the 5'-end region. The linear double-stranded DNA product may comprise a phosphorothioated nucleotide at the 5'-end or at the 5'-end region, in each strand. As most exonucleases, for example exonuclease III, remove nucleotides from the 3'-end of the polynucleotide chain, the linear double-stranded DNA product may comprise a protected nucleotide at the 3'-end or at the 3'-end region. Preferably, the linear double-stranded DNA product comprises, optionally in each strand, a phosphorothioated nucleotide at the 3'-end or the 3'-end region. The linear double-stranded DNA product may comprises at least one phosphorothioated nucleotide at the 3'-end or the 3'-end region and at least one phosphorothioated nucleotide at the 5'-end or the 5'-end region. The linear double-stranded DNA product may comprise a phosphorothioated nucleotide at the 3'-end or the 3'-end region and the 5'-end or the 5'-end region of each strand.

The phosphorothioated nucleotides might be of a different type. For example, the linear double-stranded DNA product may comprise one or more α-S-dATP (i.e. 2'-deoxyadenosine-5'-(α-thio)-triphosphate) at the 3'-end or the 3'-end region and one or more α-S-dGTP (i.e. 2'-deoxyguanosine-5'-(a-thio)-triphosphate) at the 5'-end or the 5'-end region. Alternatively, all phosphorothioated nucleotides in the linear double-stranded DNA product may be of the sample type. For example, the linear double-stranded DNA product may comprise one or more α-S-dATP at the 3'-end or the 3'-end region and one or more α-S-dATP at the 5'-end or the 5'-end region.

The linear double-stranded DNA product may be at least 100, 200, 300, 400, 500, 600, 700, 800, 900, 1000, 2000, 3000, 4000, 5000, 6000, 7000, 8000, 9000, 10000, 11000, 12000, 13000, 14000, or 15000 base pairs long. Preferably, the linear double-stranded DNA product is at least 500, or at least 1000 base pairs long.

The linear double-stranded DNA product may comprise a plurality of phosphorothioated nucleotides at internal positions in each strand. For example, the linear double-stranded DNA product may comprise at least 2, 4, 6, 8, 10, 12, 14, 16, 18, 20, 30, 40, 50, 60, 70, 80, 90, 100, 125, 150, 175, 200, 250, 300, 350, 400, 450, or 500 phosphorothioated nucleotides at internal positions in each strand. Preferably, the linear double-stranded DNA product comprises at least 2 phosphorothioated nucleotides at internal positions in each strand.

The amount of phosphorothioated nucleotides at internal positions in each strand may depend on the length of the linear double-stranded DNA product. Thus, the linear double-stranded DNA product may comprise phosphorothioated nucleotides at a ratio of the phosphorothioated nucleotides to total nucleotides that is at least 0.0001, at least 0.0025, at least 0.025, at least 0.10, at least 0.12 at least 0.15, at least 0.25, at least 0.35, at least 0.5, or at least 0.75. The linear double-stranded DNA product may comprise phosphorothioated nucleotides at a ratio of the phosphorothioated nucleotides to total nucleotides that is less than 1, less than 0.9, less than, 0.8, less than 0.65, less than 0.5, less than 0.4, less than 0.3, less than 0.2, or less than 0.1. The linear double-stranded DNA product may comprise phosphorothioated nucleotides at a ratio of the phosphorothioated nucleotides to total nucleotides that is between 0.0001-1, between 0.0025-0.75, between 0.025-0.65, between 0.025-0.15, or between 0.25-0.50. Preferably, a ratio of the phosphorothioated nucleotides to total nucleotides is between 0.025-0.15. Preferably, a ratio of the phosphorothioated nucleotides to total nucleotides is about 0.025, about 0.10, about 0.12, about 0.15, or about 0.25. More preferably still, a ratio of the phosphorothioated nucleotides to total nucleotides is about 0.025. As shown in the Examples, a ratio of the phosphorothioated nucleotides to total nucleotides of about 0.025 is sufficient to provide enhanced resistance to exonuclease digestion.

The amount of phosphorothioated nucleotides at internal positions in each strand can be different. For example, if the linear double-stranded DNA product comprises the total of 1000 nucleotides and the ratio of the phosphorothioated nucleotides to total nucleotides is 0.1, the linear double-stranded DNA product comprises 100 phosphorothioated nucleotides, out of which 75 might be located in the sense strand and 25 in the antisense strand of the DNA product. For example, 100% of the phosphorothioated nucleotides in the linear double-stranded DNA product may be located in the sense strand. Alternatively, 100% of the phosphorothioated nucleotides in the linear double-stranded DNA product may be located in the antisense strand. The sense strand may comprise at least 90%, 80%, 70%, 60%, 50%, 40%, 30%, 20%, or 10% of the total number of the phosphorothioated nucleotides in the linear double-stranded DNA product. The antisense strand may comprise at least 90%, 80%, 70%, 60%, 50%, 40%, 30%, 20%, or 10% of the total number of the phosphorothioated nucleotides in the linear double-stranded DNA product. The sense and antisense strands may comprise an equal number of the phosphorothioated nucleotides. Preferably, each of the sense and antisense strands comprises 50% of the total number of the phosphorothioated nucleotides in the linear double-stranded DNA product.

The linear double-stranded DNA product may comprise an overhang. For example, the linear double-stranded DNA product may comprise a 5' overhang or a 3' overhang. The linear double-stranded DNA product may comprise a blunt end or blunt ends. The linear double-stranded DNA product may comprise: a 5' overhang and a blunt end, two 5' overhangs, a 3' overhang and a blunt end, two 3' overhangs, or a 5' overhang and a 3' overhang.

The overhang may have at least 3 nucleotides (preferably from 4 to 8 nucleotides). The overhang may be in the sense strand or the antisense strand of the linear double-stranded DNA product.

The sense strand may have a 3'-overhang of at least one nucleotide, at least 2 nucleotides, 2 to 8 nucleotides, 2 to 7 nucleotides, 2 to 6 nucleotides, 2 to 5 nucleotides, 2 to 4 nucleotides, 2 to 3 nucleotides, 3 to 8 nucleotides, 3 to 7 nucleotides, 3 to 6 nucleotides, 3 to 5 nucleotides, 3 to 4 nucleotides, 4 to 8 nucleotides, 4 to 7 nucleotides, 4 to 6 nucleotides, 4 to 5 nucleotides, 5 to 8 nucleotides, 6 to 8 nucleotides, 6 to 7 nucleotides, or 7 to 8 nucleotides. Preferably, the sense strand has a 3'-overhang of 4 to 8 nucleotides.

The sense strand may have a 5'-overhang of at least one nucleotide, at least 2 nucleotides, 2 to 8 nucleotides, 2 to 7 nucleotides, 2 to 6 nucleotides, 2 to 5 nucleotides, 2 to 4 nucleotides, 2 to 3 nucleotides, 3 to 8 nucleotides, 3 to 7 nucleotides, 3 to 6 nucleotides, 3 to 5 nucleotides, 3 to 4 nucleotides, 4 to 8 nucleotides, 4 to 7 nucleotides, 4 to 6 nucleotides, 4 to 5 nucleotides, 5 to 8 nucleotides, 6 to 8 nucleotides, 6 to 7 nucleotides, or 7 to 8 nucleotides. Preferably, the sense strand has a 5'-overhang of 4 to 8 nucleotides.

The antisense strand may have a 3'-overhang of at least one nucleotide, at least 2 nucleotides, 2 to 8 nucleotides, 2 to 7 nucleotides, 2 to 6 nucleotides, 2 to 5 nucleotides, 2 to 4 nucleotides, 2 to 3 nucleotides, 3 to 8 nucleotides, 3 to 7 nucleotides, 3 to 6 nucleotides, 3 to 5 nucleotides, 3 to 4 nucleotides, 4 to 8 nucleotides, 4 to 7 nucleotides, 4 to 6 nucleotides, 4 to 5 nucleotides, 5 to 8 nucleotides, 6 to 8 nucleotides, 6 to 7 nucleotides, or 7 to 8 nucleotides. Preferably, the antisense strand has a 3'- overhang of 4 to 8 nucleotides.

The antisense strand may have a 5'-overhang of at least one nucleotide, at least 2 nucleotides, 2 to 8 nucleotides, 2 to 7 nucleotides, 2 to 6 nucleotides, 2 to 5 nucleotides, 2 to 4 nucleotides, 2 to 3 nucleotides, 3 to 8 nucleotides, 3 to 7 nucleotides, 3 to 6 nucleotides, 3 to 5 nucleotides, 3 to 4 nucleotides, 4 to 8 nucleotides, 4 to 7 nucleotides, 4 to 6 nucleotides, 4 to 5 nucleotides, 5 to 8 nucleotides, 6 to 8 nucleotides, 6 to 7 nucleotides, or 7 to 8 nucleotides. Preferably, the antisense strand has a 5'- overhang of 4 to 8 nucleotides.

The skilled person will appreciate that the overhangs of the sense and antisense strands may be any combination of the lengths of overhang described above. The overhangs in the linear double-stranded DNA product do not need to be of the same length. The overhangs in the linear double-stranded DNA product may be of the same length.

Each overhang and/or blunt end in the linear double-stranded DNA product may comprise one or more protected nucleotides (e.g. phosphorothioated nucleotides). The overhang and/or the blunt end of the sense strand may comprise one or more protected nucleotides. The overhang and/or the blunt end of the antisense strand may comprise one or more protected nucleotides. The 5' overhang, the 3' overhang and/or the blunt end in each strand may comprise one or more protected nucleotides. Preferably, the 5' overhang comprises one or more phosphorothioated nucleotides. Preferably, the 3' overhang comprises one or more phosphorothioated nucleotides. Preferably, the 5' overhang, the 3' overhang and/or the blunt end comprise one or more phosphorothioated nucleotides. More preferably still, each overhang and/or blunt end in the linear double-stranded DNA product comprises one or more phosphorothioated nucleotides. Each overhang and/or blunt end in the linear double-stranded DNA product may comprise at least two protected (e.g. phosphorothioated) nucleotides. The overhang and/or the blunt end of the sense strand may comprise at least two protected nucleotides. The overhang and/or the blunt end of the antisense strand may comprise at least two protected nucleotides. Each overhang and/or blunt end in the linear double-stranded DNA product may comprise at least three, four or five protected (e.g. phosphorothioated) nucleotides. The overhang and/or the blunt end of the sense strand may comprise at least three, four, or five protected nucleotides. The overhang and/or the blunt end of the antisense strand may comprise at least three, four, or five protected nucleotides. The 5' overhang, and/or the 3' overhang may comprise at least 2, 3, 4, or 5 protected nucleotides. Preferably, the 5' overhang, and/or the 3' overhang comprise at least 2 protected nucleotides, such as phosphorothioated nucleotides. More preferably still, each overhang and/or blunt end comprises at least 2 protected nucleotides, such as phosphorothioated nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

As used herein the term "protected nucleotide" or "nuclease-resistant nucleotide" is intended to encompass any type of molecule that provides or enhances resistance to nuclease digestion (especially exonuclease digestion). Although the linear double-stranded DNA product is mostly described herein as comprising phosphorothioated nucleotides, the skilled person would appreciate that the linear double-stranded DNA product may instead comprise any molecules that provide resistance to nuclease digestion (e.g. exonuclease III digestion). For example, the linear double-stranded DNA product may comprise nuclease resistant nucleotides i.e. modified nucleotides that provide or increase resistance to nucleases (e.g. exonucleases). The linear double-stranded DNA product may comprise a peptide, polypeptide or protein that provides or increases resistance to nucleases (e.g. exonucleases) digestion. The linear double-stranded DNA product may comprise 2'-O-methyl nucleotides or 2'-O-methoxyethyl (MOE) nucleotides.

The invention provides a linear double-stranded DNA product comprising a sense strand and an antisense strand, wherein the linear double-stranded DNA product comprises a single cassette and at least two MOE nucleotides at internal positions in each strand, wherein the at least two MOE nucleotides at internal positions in each strand are selected from:
(a) a 5'-end nucleotide of the cassette;
(b) a 3'-end nucleotide of the cassette; and
(c) one or more nucleotides outside of the cassette; and

wherein the cassette comprises a coding sequence.

The nucleotides resistant to exonuclease digestion (i.e. protected nucleotides) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate, preferably α-S-dATP, α-S-dGTP or α-S-dTTP.

The linear double-stranded DNA product may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The linear double-stranded DNA product may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The linear double-stranded DNA product may comprise at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The amount of incorporation of the protected nucleotides in the linear double-stranded DNA product may be determined based on methods known in the art. For example, a primer extension assays may be performed to assess the incorporation rate of protected versus unprotected nucleotides. For example, the single-nucleotide extension of a template/primer molecule by polymerase may be evaluated in the presence of increasing concentrations of protected and unprotected complementary nucleotides to the first base of the template. The efficiency of incorporation under linear (non-saturated) conditions may be used to determine the degree of incorporation of protected nucleotides in comparison to natural unprotected nucleotides.

### 2. Complex molecules

The linear double-stranded DNA product may further comprise a functional portion.

The invention provides a complex molecule comprising the linear double-stranded DNA product described herein and a functional portion. Optionally, the functional portion is a binding molecule or a probe.

The invention provides a complex molecule comprising:
(a) a linear double-stranded DNA product comprising a sense strand and an antisense strand, wherein the linear double-stranded DNA product comprises a single cassette and one or more phosphorothioated nucleotides at internal positions in each strand; and
(b) a functional portion.

Preferably, the one or more phosphorothioated nucleotides at the internal positions in each strand may be selected from:
i. a 5'-end nucleotide of the cassette;
ii. a 3'-end nucleotide of the cassette; and
iii. one or more nucleotides outside of the cassette.

The invention also provides a complex molecule comprising:
(a) a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
   i. a 5'-end nucleotide of the cassette;
   ii. a 3'-end nucleotide of the cassette; and
   iii. one or more nucleotides outside of the cassette; and
   wherein the cassette comprises a coding sequence; and
(b) a functional portion.

The functional portion may be at the 3' and/or the 5' end (or end region) of the polynucleotide chain. The functional portion may be attached or bound to the 5' overhang, the 3' overhang and/or the blunt end of the linear double-stranded DNA product. The functional portion may be attached or bound to the 5' overhang, the 3' overhang and/or the blunt end of the linear double-stranded DNA product by covalent linkage or non-covalent linkage, or by nucleic acid hybridisation. The functional portion may be attached or bound to the linear double-stranded DNA product directly or indirectly (e.g. via a linker molecule). The functional portion may be attached or bound by being bound to the linear double-stranded DNA product and/or by being bound or annealed to linker molecules that are bound to the linear double-stranded DNA product. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol).

The linear double-stranded DNA product may comprise two functional portions; a first functional portion at the 3'-end of the polynucleotide chain, and a second functional portion at the 5'-end of the polypeptide chain. The first functional portion may be attached or bound to the 5' overhang, the 3' overhang and/or the blunt end of the 3'-end of the linear double-stranded DNA product. The second functional portion may be attached or bound to the 5' overhang, the 3' overhang and/or the blunt end of the 5'-end of the linear double-stranded DNA product. The two functional portions may be the same or different. For example, the first functional portion may be a barcode to facilitate detection and/or sequencing of the DNA product, the second functional portion may be a nuclear targeting sequence.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear double-stranded DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. Thus, the sense strand of the linear double-stranded DNA product of the invention may comprise a label at the 5'-end or the 3'-end for detection. Alternatively or additionally, the antisense strand of the linear double-stranded DNA product of the invention may comprise a label at the 5'-end or the 3'-end for detection. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the DNA product. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the DNA product, the functional portion may comprise a fluorophore, a radioactive compound or a barcode.

A signal corresponding to the presence, absence and/or level of the linear double-stranded DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the linear double-stranded DNA product. The binding moiety is capable of binding to 3' and/or 5' end of the linear double-stranded DNA product. The signal may be measured by determining the presence, absence and/or level of the barcoded portion of the barcode (e.g. by sequencing or PCR). The barcoded portion may comprise at least 2, 3, 4, 5, 6, 7, 8, 9, or 10 nucleotides. The barcode may comprise at least 2 binding moieties (e.g. a first binding moiety and a second binding moiety). For example, the first binding moiety linked to the first barcoded portion may bind to the 3' end of the linear double-stranded DNA product and the second binding moiety linked to the second barcoded portion may bind to the 5' end of the linear double-stranded DNA product. The 3' and 5' ends may comprise a 3' overhang, a 5' overhang or a blunt end.

A signal corresponding to the presence, absence and/or level of the DNA product may be measured using a fluorophore (i.e. a fluorescently-labelled molecule), which is attached or bound to the 3' overhang, the 5' overhang or the blunt end of the linear double-stranded DNA product. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

### 3. Delivery particles

Any or all of the linear double-stranded DNA products or complex molecules may be delivered to a cell via delivery particles, such as nanoparticles.

The invention provides a nanoparticle (or a library of nanoparticles) that is particularly suitable for use in gene therapies. The invention provides the linear double-stranded DNA product, the complex molecule, the nanoparticle, the composition, or the library for use in gene therapy.

The nanoparticle (or a library of nanoparticles) can be used to treat diseases by delivering DNA products encoding therapeutic proteins or by delivering DNA products useful in gene-editing (e.g. genes of the CRISPR system). Thus, the nanoparticle may function as a delivery system for various applications. The delivery may be in vitro or in vivo.

Effective cellular internalization of therapeutic genes is a critical process for the successful clinical application of nanoparticles for gene delivery. Thus, the invention provides a nanoparticle (or a library of nanoparticles) for cellular internalization.

The invention provides a nanoparticle comprising the linear double-stranded DNA product, the complex molecule or a library described herein. Thus, the invention provides a nanoparticle comprising a linear double-stranded DNA product comprising a sense strand and an antisense strand, wherein the linear double-stranded DNA product comprises a single cassette and one or more phosphorothioated nucleotides at internal positions in each strand. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention also provides a nanoparticle comprising a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The invention also provides a library of nanoparticles, wherein each nanoparticle comprises a linear double-stranded DNA product and wherein the linear double-stranded DNA products have low dispersity. The term "low dispersity" in the context of nanoparticles (or a library of nanoparticles) is intended to encompass linear double-stranded DNA products that have substantially the same size (i.e. number of bases in the polynucleotide chain). That is to say, each linear double-stranded DNA product may vary in size (i.e. number of bases in the polynucleotide chain) from the other linear double-stranded DNA products in the library of nanoparticles by less than 30%, 20%, or 10%, preferably by less than 5%. These DNA products result in the formation of uniform nanoparticles or a collection (i.e. library) of uniform nanoparticles. Conversely, nanoparticles comprising a polydisperse DNA product (for example, such that produced by a method described in US Patent US10350307 B2) would be of different sizes and shapes (i.e. higher dispersity). Uniformity (and/or low dispersity) of nanoparticles is particularly beneficial for transformation and/or transfection and play a key role in internalization and drug release processes.

The invention also provides a library of nanoparticles comprising a library of linear double-stranded DNA products as described herein.

The nanoparticle may comprise at least one copy of the linear double-stranded DNA product. The nanoparticle may comprise at least 2, 3, 4, 5, 6, 7, 8, 9, 10 copies of the linear double-stranded DNA product. Each nanoparticle in the library of nanoparticles may comprise at least one copy of the linear double-stranded DNA product. Each nanoparticle in the library of nanoparticles may comprise at least 2, 3, 4, 5, 6, 7, 8, 9, 10 copies of the linear double-stranded DNA product. Each nanoparticle in the library of nanoparticles may comprise the same amount of copies of the linear double-stranded DNA product. Each nanoparticle in the library of nanoparticles may comprise 3 copies of the linear double-stranded DNA product. Each nanoparticle in the library of nanoparticles may comprise 4 copies of the linear double-stranded DNA product. Each nanoparticle in the library of nanoparticles may not comprise more than 10 copies of the linear double-stranded DNA product. Each nanoparticle in the library of nanoparticles may not comprise a concatemeric DNA product. Each nanoparticle in the library of nanoparticles may not comprise a linear double-stranded DNA product comprising a plurality of cassettes.

The nanoparticle may comprise at least one copy of the complex molecule. The nanoparticle may comprise at least 2, 3, 4, 5, 6, 7, 8, 9, 10 copies of the complex molecule. Each nanoparticle in the library of nanoparticles may comprise at least one copy of the complex molecule. Each nanoparticle in the library of nanoparticles may comprise at least 2, 3, 4, 5, 6, 7, 8, 9, 10 copies of the complex molecule. Each nanoparticle in the library of nanoparticles may comprise the same amount of copies of the complex molecule. Each nanoparticle in the library of nanoparticles may comprise 3 copies of the complex molecule. Each nanoparticle in the library of nanoparticles may comprise 4 copies of the complex molecule. Each nanoparticle in the library of nanoparticles may not comprise more than 10 copies of the complex molecule.

The library may comprise at least 2, 3, 4, 5, 10, 15, 20, 30, 40, 50, 60, 70, 80, 90, 100, 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, or 10¹⁷ nanoparticles. For example, the library may comprise at least 2, 3, 4, 5, 10, 15, 20, 30, 40, 50, 60, 70, 80, 90, 100, 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, or 10¹⁷ nanoparticles per mL.

The nanoparticles may be self-assembling nanoparticles. Preferably, the nanoparticles may be particles that can be made on a microfluidics devices. Preferably, the nanoparticles assemble based on the electrostatic interactions, e.g. between the natively charged DNA molecule and a positively charged cationic lipid or peptide. For example, a nanoparticle may be based on self-assembling bioadhesive polymers, which may be applied to oral delivery, intravenous delivery and/or nasal delivery of linear double-stranded DNA products. For example, a nanoparticle may deliver the DNA product to a cancer cell to stop tumour growth.

The nanoparticle may be produced by methods known in the art. For example, the nanoparticle may be produced by encapsulation of the linear double-stranded DNA product described herein or the complex molecule described herein in poly(lactic-co-glycolic acid) nanoparticles by double-emulsion solvent evaporation method. The nanoparticle may be further modified with a cationic component, for example biocompatible chitosan or cyclodextrin.

The nanoparticle may be produced by ethanol loading formation process. In this process, lipids dissolved in ethanol are rapidly mixed with the linear double-stranded DNA product described herein or the complex molecule described herein in aqueous buffer of acidic pH (preferably pH=4). In this self-assembly process, the electrostatic interactions drive formation of an inverted micelle comprising or essentially consisting of the DNA product or the complex molecule.

The nanoparticle may be a lipid-based nanoparticle, a LNP (a lipid nanoparticle), a polymeric nanoparticle, a PEI-based nanoparticle, a lipopolyplex nanoparticle (mixture of polymers and lipids), or a solid nanoparticle (e.g. a gold nanoparticle).

Preferably, the nanoparticle (or a library of nanoparticles) is capable of transfecting cells as described herein. Preferably, the cells are human cells.

The nanoparticles (or libraries of nanoparticles) described herein are suitable for use in gene therapy. For example, the nanoparticles (or libraries of nanoparticles) can be used for a controlled gene delivery process. The nanoparticle may be a gold nanoparticle, a nanodiamond, or a porous silicon nanoparticle. The nanoparticle suitable for gene therapy may be any type of nanoparticle that has a good stability, easy surface functionalization, and/or easy size control. Gold nanoparticles are particularly suitable due to their nontoxic properties. The nanoparticle may be a lipid-based nanoparticle.

The lipid-based nanoparticles are particularly suitable for gene delivery due to their high biocompatibility and close resemblance to the lipid membranes. The lipid-based nanoparticles typically have improved cell penetration comparing to other known nanoparticles. The nanoparticle may be a large unilamellar vesicle nanoparticle. For example, LNPs have been shown to be highly effective in delivering siRNAs to the liver (Tabernero et al., Cancer Discovery, April 2013, Vol. 3, No. 4, pages 363-470) and are therefore contemplated for delivering the DNA products and complex molecules described herein to the liver.

US patent application 20110293703 relates to lipidoid compounds that are useful in the administration of polynucleotides, which may be applied to deliver the DNA products and complex molecules of the present invention. For example, the aminoalcohol lipidoid compounds are combined with an agent to be delivered to a cell or a subject to form microparticles, nanoparticles, liposomes, or micelles. The agent to be delivered by the microparticles, nanoparticles, liposomes, or micelles may be in the form of a gas, liquid, or solid, and the agent may be a polynucleotide, protein, peptide, or small molecule. The aminoalcohol lipidoid compounds may be combined with other aminoalcohol lipidoid compounds, polymers, surfactants, cholesterol, proteins, lipids, etc. to form the nanoparticles. These nanoparticles may then optionally be combined with a pharmaceutical excipient to form a pharmaceutical composition.

Spherical Nucleic Acid (SNA) constructs and other nanoparticles (e.g. gold nanoparticles) are also suitable as a means to delivery the DNA product and/or complex molecules to intended targets. Significant data show that AuraSense Therapeutics' Spherical Nucleic Acid (SNA^{™}) constructs, based upon nucleic acid-functionalized gold nanoparticles, are superior to alternative platforms based on multiple key success factors, such as: 1) high in vivo stability (due to their dense loading, a majority of cargo (DNA product or complex molecule) remains bound to the constructs inside cells, conferring nucleic acid stability and resistance to enzymatic degradation); 2) deliverability (for all cell types studied (e.g. neurons, tumour cell lines, etc.) the constructs demonstrate a transfection efficiency of 99% with no need for carriers or transfection agents); 3) therapeutic targeting (the unique target binding affinity and specificity of the constructs allow high specificity for matched target sequences); 4) superior efficacy; 5) low toxicity; 6) no significant immune response; and 7) chemical tailorability.

Self-assembling nanoparticles with the DNA products or complex molecules may be constructed with polyethyleneimine (PEI) that is PEGylated with an Arg-Gly-Asp (RGD) peptide and attached at the distal end of the polyethylene glycol (PEG). Nanoplexes may be prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid may result in the formation of polyplexes with average particle size distribution of about 100 nm, hence referred to here as nanoplexes.

A nanoparticle may comprise a biocompatible nanocarrier. For example, the nanoparticle may comprise poly(β-amino ester)s, low-molecular-weight polyethylenimine, polyphosphoesters, disulfide coss-linked polymers and polyamidoamine. These nanocarriers show improved nanoparticle formulation stability and safety over conventional gene delivery mechanisms.

The nanoparticle may be linked to additional components. For example, the additional components might ensure specificity and selectivity of nanoparticles in gene therapy. For example, a nanoparticle may be linked with a peptide. The peptide may improve the targeting ability of the nanoparticle, reduce the biological toxicity and improve the therapeutic effect. The nanoparticle may be attached to a liposome, preferably, a fusogenic liposome. The nanoparticle may be additionally or alternatively attached to an outer sheath of a homing peptide.

The nanoparticle may be a magnetic nanoparticle. The nanoparticle may be a peptide-based nanoparticle. The nanoparticle may be a part of a nanomaterial suitable for gene therapy. For example the nanomaterial may comprise a small molecule, a nanoparticle and a polymer.

A nanoparticle may comprise the linear double-stranded DNA product described here, which is attached or bound to a functional portion. Thus, the nanoparticle may comprise a complex molecule as described herein. The functional portion of the complex molecule may facilitate detection of nanoparticle. The detection may be performed in vivo or in vitro. For example, the functional portion may be a fluorescence label, which may be tracked in vivo or imaged in vitro.

The functional portion may be a probe or a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the DNA product.

The functional portion may be a binding molecule. The functional portion may be a protein or a peptide. The functional portion may be an antibody, or an enzyme. The functional portion may facilitate detection of the nanoparticle by binding to capture molecules (e.g. capture antibodies bound by protein-protein interactions). The functional portion may bind to a cell target, for example, a cell receptor.

To facilitate detection and/or quantification of the nanoparticle, the functional portion may comprise a fluorophore, a radioactive compound or a barcode.

A signal corresponding to the presence, absence and/or level of the linear double-stranded DNA product in a nanoparticle may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the linear double-stranded DNA product. The binding moiety is capable of binding to 3' and/or 5' end of the linear double-stranded DNA product. The signal may be measured by determining the presence, absence and/or level of the barcoded portion of the barcode (e.g. by sequencing or PCR). The barcoded portion may comprise at least 2, 3, 4, 5, 6, 7, 8, 9, or 10 nucleotides. The barcode may comprise at least 2 binding moieties (e.g. a first binding moiety and a second binding moiety). For example, the first binding moiety linked to the first barcoded portion may bind to the 3' end of the linear double-stranded DNA product and the second binding moiety linked to the second barcoded portion may bind to the 5' end of the linear double-stranded DNA product. The 3' and 5' ends may comprise a 3' overhang, a 5' overhang or a blunt end.

A signal corresponding to the presence, absence and/or level of the DNA product in a nanoparticle may be measured using a fluorophore (i.e. a fluorescently-labelled molecule), which is attached or bound to the 3' overhang, the 5' overhang or the blunt end of the linear double-stranded DNA product. The signal may be measured by flow cytometry and/or fluorescence-activated cell sorting.

### 4. Libraries

The invention provides a library comprising at least two linear double-stranded DNA products or at least two complex molecules described herein. Thus, the invention provides a library comprising at least two linear double-stranded DNA products, each of which comprising a sense strand and an antisense strand, wherein each linear double-stranded DNA product comprises a single cassette and one or more phosphorothioated nucleotides at internal positions in each strand. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention provides a library comprising at least two linear double-stranded DNA products, each of which comprising a sense strand and an antisense strand, wherein each linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The library may comprise at least 2, 3, 4, 5, 10, 15, 20, 30, 40, 50, 60, 70, 80, 90, 100, 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, or 10¹⁷ linear double-stranded DNA products.

For example, the library may comprise at least 2, 3, 4, 5, 10, 15, 20, 30, 40, 50, 60, 70, 80, 90, 100, 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, or 10¹⁷ linear double-stranded DNA products per mL. Preferably, the library comprises at least 10¹⁰ linear double-stranded DNA products (or molecules). For example, the library comprises at least 10¹⁰ linear double-stranded DNA products (or molecules) per mL. The library may comprise between 10⁶ - 10¹⁶ linear double-stranded DNA products, preferably between 10¹³-10¹⁶ linear double-stranded DNA products. The library may comprise at least 2, 3, 4, 5, 10, 15, 20, 30, 40, 50, 60, 70, 80, 90, 100, 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, or 10¹⁷ complex molecules. For example, the library may comprise at least 2, 3, 4, 5, 10, 15, 20, 30, 40, 50, 60, 70, 80, 90, 100, 10³, 10⁴, 10⁵, 10⁶, 10⁷, 10⁸, 10⁹, 10¹⁰, 10¹¹, 1012, 10¹³, 10¹⁴, 10¹⁵, 10¹⁶, or 10¹⁷ complex molecules per mL. Preferably, the library comprises at least 10¹⁰ complex molecules. For example, the library comprises at least 10¹⁰ complex molecules per mL. The library may comprise between 10⁶ - 10¹⁶ complex molecules, preferably between 10¹³-10¹⁶ complex molecules. Each linear double-stranded DNA product or complex molecule may encode a different gene of interest or the same gene of interest (or a collection of the same genes of interest).

The library of linear double-stranded DNA products or complex molecules may be monodisperse. The library of linear double-stranded DNA products or complex molecules may have low dispersity. The terms "monodisperse" and "low dispersity" in the context of libraries is intended to encompass linear double-stranded DNA products or complex molecules that have substantially the same size (i.e. number of bases in the polynucleotide chain). The monodisperse library (or the library of low dispersity) of DNA products or complex molecules may be used, for example, in the formation of nanoparticles. This is because the lower the dispersity of the starting material (i.e. linear double-stranded DNA products or complex molecules), the lower the dispersity (and higher the uniformity) of the nanoparticles produced.

In the library, each linear double-stranded DNA product may vary in size (i.e. number of bases in the polynucleotide chain) from the other linear double-stranded DNA products in the library by less than 30%, 20%, or 10%, preferably by less than 5%. In the library, each linear double-stranded DNA product may vary in size (i.e. number of bases in the polynucleotide chain) from at least 10¹⁰ other linear double-stranded DNA products in the library by less than 30%, 20%, or 10%. Preferably, in the library, each linear double-stranded DNA product varies in size (i.e. number of bases in the polynucleotide chain) from at least 10¹⁰ other linear double-stranded DNA products in the library by less than 5%.

In the library, each complex molecule may vary in size (i.e. number of bases in the polynucleotide chain) from the other complex molecules in the library by less than 30%, 20%, or 10%, preferably by less than 5%. In the library, each complex molecule may vary in size (i.e. number of bases in the polynucleotide chain) from at least 10¹⁰ other complex molecules in the library by less than 30%, 20%, or 10%. Preferably, in the library, each complex molecule varies in size (i.e. number of bases in the polynucleotide chain) from at least 10¹⁰ other complex molecules in the library by less than 5%.

In the library, each linear double-stranded DNA product may vary in size (i.e. number of bases in the polynucleotide chain) from the other linear double-stranded DNA products in the library by less than 1000, 500, 250, 100, or 50 nucleotides, preferably by less than 50 nucleotides. In the library, each linear double-stranded DNA product may vary in size (i.e. number of bases in the polynucleotide chain) from at least 10¹⁰ other linear double-stranded DNA products in the library by less 500 nucleotides. In the library, each linear double-stranded DNA product may vary in size (i.e. number of bases in the polynucleotide chain) from at least 10¹⁰ other linear double-stranded DNA products in the library by less 250 nucleotides. In the library, each linear double-stranded DNA product may vary in size (i.e. number of bases in the polynucleotide chain) from at least 10¹⁰ other linear double-stranded DNA products in the library by less 100 nucleotides. Preferably, in the library, each linear double-stranded DNA product varies in size (i.e. number of bases in the polynucleotide chain) from at least 10¹⁰ other linear double-stranded DNA products in the library by less than 50 nucleotides.

In the library, each complex molecule may vary in size (i.e. number of bases in the polynucleotide chain) from the other complex molecules in the library by less than 1000, 500, 250, 100, or 50 nucleotides, preferably by less than 50 nucleotides. In the library, each complex molecule may vary in size (i.e. number of bases in the polynucleotide chain) from at least 10¹⁰ other complex molecules in the library by less 500 nucleotides. In the library, each complex molecule may vary in size (i.e. number of bases in the polynucleotide chain) from at least 10¹⁰ other complex molecules in the library by less 250 nucleotides. In the library, each complex molecules may vary in size (i.e. number of bases in the polynucleotide chain) from at least 10¹⁰ other complex molecules in the library by less 100 nucleotides. Preferably, in the library, each complex molecules varies in size (i.e. number of bases in the polynucleotide chain) from at least 10¹⁰ other complex molecules in the library by less than 50 nucleotides.

Most of the linear double-stranded DNA products or complex molecules in the library may have substantially the same size (i.e. number of bases in the polynucleotide chain). That is to say that the library may comprise at least 80%, 85%, 90%, 95%, or 100% of the linear double-stranded DNA products that are of substantially the same size (i.e. number of bases in the polynucleotide chain). For example, in a library of 10¹⁰ linear double-stranded DNA products, at least 90% (i.e. at least 10⁹) of the linear double-stranded DNA products may be of substantially the same size (i.e. number of bases in the polynucleotide chain). The library may comprise at least 80%, 85%, 90%, 95%, or 100% of the complex molecules that are of substantially the same size (i.e. number of bases in the polynucleotide chain). For example, in a library of 10¹⁰ complex molecules, at least 90% (i.e. at least 10⁹) of complex molecules may be of substantially the same size (i.e. number of bases in the polynucleotide chain).

In the library, each linear double-stranded DNA product may vary in size (i.e. number of bases in the polynucleotide chain) from at least 80%, 85%, 90%, 95% or 100% of the other linear double-stranded DNA products in the library by less than 30%, 20%, or 10%, preferably by less than 5%. In the library, each linear double-stranded DNA product may vary in size (i.e. number of bases in the polynucleotide chain) from at least 90% of the other linear double-stranded DNA products in the library by less than 10%. Preferably, in the library, each linear double-stranded DNA product varies in size (i.e. number of bases in the polynucleotide chain) from at least 90% of the other linear double-stranded DNA products in the library by less than 5%.

In the library, each complex molecule may vary in size (i.e. number of bases in the polynucleotide chain) from at least 80%, 85%, 90%, 95% or 100% of the other complex molecules in the library by less than 30%, 20%, or 10%, preferably by less than 5%. In the library, each complex molecule may vary in size (i.e. number of bases in the polynucleotide chain) from at least 90% of the other complex molecules in the library by less than 10%. Preferably, in the library, each complex molecule varies in size (i.e. number of bases in the polynucleotide chain) from at least 90% of the other complex molecules in the library by less than 5%.

The linear double-stranded DNA products or complex molecules of a library may be substantially identical. That is to say that each linear double-stranded DNA product or complex molecule in the library is substantially the same, i.e. comprises the same, or substantially the same polynucleotide sequence. For example, the library may comprise linear double-stranded DNA products with at least 80%, 85%, 90%, 92%, 94%, 96%, 97%, 98%, 99%, or 100% sequence similarity. For example, the library may comprise complex molecules with at least 80%, 85%, 90%, 92%, 94%, 96%, 97%, 98%, 99%, or 100% sequence similarity. For example, the library may comprise linear double-stranded DNA products with at least 80%, 85%, 90%, 92%, 94%, 96%, 97%, 98%, 99%, or 100% sequence identity. For example, the library may comprise complex molecules with at least 80%, 85%, 90%, 92%, 94%, 96%, 97%, 98%, 99%, or 100% sequence identity. Preferably, the library comprises linear double-stranded DNA products with at least 95% sequence similarity. More preferably still, the library comprises linear double-stranded DNA products with at least 95% sequence identity. Preferably, the library comprises complex molecules with at least 95% sequence similarity. More preferably still, the library comprises complex molecules with at least 95% sequence identity.

Although each linear double-stranded DNA product or complex molecule in the library may be substantially the same (i.e. may comprise the same, or substantially the same polynucleotide sequence), the location and/or distribution of protected nucleotides (e.g. phosphorothioated nucleotides) in some (or all) linear double-stranded DNA products or complex molecules in the library may be different.

In the library of linear double-stranded DNA products, at least 80%, 85%, 90%, 95%, 96%, 97%, 98%, 99% or 100% of the linear double-stranded DNA products may have a different distribution of protected nucleotides (e.g. phosphorothioated nucleotides). Preferably, at least 99% of the linear double-stranded DNA products in the library have a different distribution of protected nucleotides (e.g. phosphorothioated nucleotides). That is to say that at least 80%, 85%, 90%, 95%, 96%, 97%, 98%, 99% or 100% of the linear double-stranded DNA products in the library may not have the same distribution of protected nucleotides (e.g. phosphorothioated nucleotides) as any other linear double-stranded DNA product in the library. Preferably, at least 99% of the linear double-stranded DNA products in the library do not have the same distribution of protected nucleotides (e.g. phosphorothioated nucleotides) as any other linear double-stranded DNA product in the library.

In the library of complex molecules, at least 80%, 85%, 90%, 95%, 96%, 97%, 98%, 99% or 100% of the complex molecules may have a different distribution of protected nucleotides (e.g. phosphorothioated nucleotides). Preferably, at least 99% of the complex molecules in the library have a different distribution of protected nucleotides (e.g. phosphorothioated nucleotides). That is to say that at least 80%, 85%, 90%, 95%, 96%, 97%, 98%, 99% or 100% of the complex molecules in the library may not have the same distribution of protected nucleotides (e.g. phosphorothioated nucleotides) as any other complex molecule in the library. Preferably, at least 99% of the complex molecules in the library do not have the same distribution of protected nucleotides (e.g. phosphorothioated nucleotides) as any other complex molecule in the library.

The expression "different distribution of protected nucleotides" as used herein is meant to encompass linear double-stranded DNA products or complex molecules that do not have identical distribution of all protected nucleotides. For example, a molecule comprising protected nucleotides at locations 2, 3, 7, and 8, has a different distribution of protected nucleotides to a molecule comprising protected nucleotides at locations 2, 3, 7, and 9.

Each linear double-stranded DNA product in the library may be at least 100, 200, 300, 400, 500, 600, 700, 800, 900, 1000, 2000, 3000, 4000, 5000, 6000, 7000, 8000, 9000, 10000, 11000, 12000, 13000, 14000, or 15000 base pairs long. Preferably, each linear double-stranded DNA product in the library is at least 500, or at least 1000 base pairs long.

Each complex molecule in the library may be at least 100, 200, 300, 400, 500, 600, 700, 800, 900, 1000, 2000, 3000, 4000, 5000, 6000, 7000, 8000, 9000, 10000, 11000, 12000, 13000, 14000, or 15000 base pairs long. Preferably, each complex molecule in the library is at least 500, or at least 1000 base pairs long.

The functional portion of the complex molecules in the library may be the same or different. For example, the library may comprise complex molecules with different functional portions and at least 80%, 85%, 90%, 92%, 94%, 96%, 97%, 98%, 99%, or 100% sequence identity or similarity in respect of the sequence of the linear double-stranded DNA product part of the complex molecules.

A library of the invention may be useful for the screening of gene function in cellular processes as well as diseases. The library may be useful for gene sequencing.

The invention also provides a library comprising a population of transfected cells produced by the methods described herein. The cells may comprise the linear double-stranded DNA product or the complex molecule of the invention. For example, the cells may comprise a linear double-stranded DNA product comprising a sense strand and an antisense strand, wherein the linear double-stranded DNA product comprises a single cassette and one or more phosphorothioated nucleotides at internal positions in each strand.

The cells may comprise a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

### 5. Compositions

The invention further provides a composition comprising a linear double-stranded DNA product, a complex molecule, a nanoparticle, or a library described herein. The composition may be a pharmaceutical composition. The composition or pharmaceutical composition may further comprise a pharmaceutically acceptable diluent.

The invention provides a composition comprising the linear double-stranded DNA product described herein. Thus, the invention provides a composition comprising a linear double-stranded DNA product comprising a sense strand and an antisense strand, wherein the linear double-stranded DNA product comprises a single cassette and one or more phosphorothioated nucleotides at internal positions in each strand. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention also provides a composition comprising a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The pharmaceutically acceptable diluent may be saline, a buffered solution (e.g. a buffered aqueous solution) or another excipient.

For instance, composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The composition may be administered orally, topically, parenterally or transdermally or by inhalation. The composition may be administered by injection or intravenous infusion using suitable sterile solutions. Topical dosage forms may be creams, ointments, patches, or similar vehicles suitable for transdermal and topical dosage forms.

The composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the linear double-stranded DNA product, the complex molecule, the nanoparticle, or the library described herein can he administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochiear capsule.

Other forms include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the composition can be administered in situ, via a catheter or pump. A catheter or pump can, for example, direct the composition into the target location.

The parenteral carrier system includes one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

As used herein, transdermal dosage form includes, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

As used herein, the topical dosage form includes various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 6. Cell transfection

The invention provides a cell transfection composition comprising a linear double-stranded DNA product or a complex molecule of the invention.

The invention provides a cell transfection composition comprising the linear double-stranded DNA product described herein. Thus, the invention provides a cell transfection composition comprising a linear double-stranded DNA product comprising a sense strand and an antisense strand, wherein the linear double-stranded DNA product comprises a single cassette and one or more phosphorothioated nucleotides at internal positions in each strand. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention also provides a cell transfection composition comprising a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

The cell transfection composition may or may not comprise a carrier e.g. an agent or a formulation. Preferably, the carrier promotes accumulation of the linear double-stranded DNA product or the complex molecule at a target site and/or protects the linear double-stranded DNA product or the complex molecule from undesirable interactions with biological milieu components and/or protects the linear double-stranded DNA product or the complex molecule from metabolism and/or degradation.

The invention further provides a cell transfection method comprising contacting (in vitro) a cell to be transfected with a linear double-stranded DNA product, a complex molecule, a nanoparticle, or a library of the invention, and wherein the linear double-stranded DNA product, the complex molecule, the nanoparticle, or the library is transfected into the cytosol of the cell.

The cell may be contacted with the linear double-stranded DNA product, the complex molecule, the nanoparticle, or the library in the presence or absence of a carrier e.g. an agent or a formulation. Preferably, the carrier promotes accumulation of the linear double-stranded DNA product, the complex molecule, the nanoparticle, or the library at a target site and/or protects the linear double-stranded DNA product, the complex molecule, the nanoparticle, or the library from undesirable interactions with biological milieu components and/or protects the linear double-stranded DNA product, the complex molecule, the nanoparticle, or the library from metabolism and/or degradation.

The cell or cells to be transfected may be provided in a cell culture medium (e.g. in a Petri dish, culture vessel or well, etc). The linear double-stranded DNA product, the complex molecule, the nanoparticle, or the library molecule may be added directly to the cell culture medium or the cells may be added to a solution, such as saline, a buffered solution or a cell culture medium, comprising the linear double-stranded DNA product, the complex molecule, the nanoparticle, or the library.

The carrier may be a viral carrier or a non-viral carrier. Viral carriers include a lentiviral vector or an adenoviral vector for delivery of the linear double-stranded DNA product or the complex molecule. Non-viral carriers (or vectors) include complexing the linear double-stranded DNA product or the complex molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the linear double-stranded DNA product or the complex molecule. The carrier may be a nanoparticulate formulation used to encapsulate the linear double-stranded DNA product or the complex molecule.

The carrier may be a modification of the linear double-stranded DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the linear double-stranded DNA product or the complex molecule at a target site and/or protects the linear double-stranded DNA product or the complex molecule from metabolism and/or degradation.

The invention further provides a cell obtainable by the methods of the invention. Thus, the cell may comprise a linear double-stranded DNA product, a complex molecule, a nanoparticle, or a library of the invention.

The invention further provides a cell transfected with a linear double-stranded DNA product, a complex molecule, a nanoparticle, or a library of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a linear double-stranded DNA product, a complex molecule, a nanoparticle, or a library may be performed in vivo. For example the linear double-stranded DNA product, the complex molecule, the nanoparticle, or the library may be administered to an organism (e.g. a subject) in need thereof. The organism (e.g. the subject) may be an animal, such as mammal (e.g. a human), a fungus, a micro-organism, or a plant.

Any or all of the linear double-stranded DNA products or complex molecules described herein may be delivered to a cell via delivery particles such as liposomes, nanoparticles, exosomes, macrovesicles, viral or non-viral vectors. Any or all of the linear double-stranded DNA products or complex molecules described herein may be delivered to a cell using a gene-gun. The linear double-stranded DNA products or complex molecules described herein may be delivered to a cell without a carrier.

### 7. Uses and applications

The linear double-stranded DNA product of the present invention has many benefits over the amplification products known in the art. For example, unlike plasmid DNAs that are typically used for transcription of the gene of interest, the linear double-stranded DNA product does not comprise a bacterial backbone, antibiotic resistant genes and bacterial contaminants. In addition, the linear double-stranded DNA product can be produced in large quantity in a cell-free system, which substantially expedites the manufacturing process. Importantly, the linear double-stranded DNA product may comprise a single cassette, which may comprise a coding sequence. This means that the generated product has low dispersity (or is substantially monodisperse), i.e. is of substantially the same length and size, which is highly beneficial in the production of nanoparticles, viral or non-viral delivery systems and vaccines. The linear double-stranded DNA product may also have an improved resistance to exonuclease digestion, which provides a longer half-life of the product (and prolonged in vivo expression). For the reasons discussed above, the linear double-stranded DNA product described herein is suitable for many applications, some of which are discussed herein.

Uses of the products comprising the protected nucleotides described herein may be *in vivo* or *in vitro* uses.

### Production of RNA and protein

The invention provides the use of the linear double-stranded DNA product, the complex molecule, the nanoparticle, or the library in the production of RNA (e.g. mRNA).

The invention provides a method for in vitro transcription, comprising:
(a) contacting the linear double-stranded DNA product described herein with a polymerase; and
(b) producing a transcription product.

The invention provides a method for in vitro transcription, comprising (a) contacting the complex molecule described herein with a polymerase; and (b) producing a transcription product. The invention provides a method for in vitro transcription, comprising (a) contacting the nanoparticle described herein with a polymerase; and (b) producing a transcription product. The invention also provides a method for in vitro transcription, comprising (a) contacting the library described herein with a polymerase; and (b) producing a transcription product.

The invention also provides a method for in vivo transcription, comprising (a) contacting the linear double-stranded DNA product described herein with a polymerase in a cell; and (b) producing a transcription product in the cell.

The cell may be an animal cell, preferably a mammal cell, such as a human cell.

The linear double-stranded DNA product may comprise a single cassette. The cassette may comprise a coding sequence. The cassette may comprise a promoter and a coding sequence. The coding sequence may encode a desired protein. Thus, the transcription product may comprise an mRNA sequence of a desired protein.

The transcription may comprise the use of a polymerase. Preferably, an RNA polymerase. The RNA polymerase may be T7 RNA polymerase, T3 RNA polymerase, or SP6 RNA polymerase.

The transcription may comprise a step of incorporation of protected nucleotides, such as phosphorothioated nucleotides.

The in vitro transcription may be performed in a cell-free expression system. The cell-free expression system may originate from a prokaryotic cell or eukaryotic cell. For example, the cell-free expression system may originate from rabbit reticulocytes, wheat germ or *Escherichia coli.*

The transcription product may be used in viral RNA synthesis and transfection. For example, the transcription product may be used to start replication. The transcription product may be used in in vitro and/or in vivo mRNA synthesis. The transcription product may be used in RNA structure studies. The transcription product may be used in RNA aptamer synthesis, for example, for systematic evolution of ligands by exponential enrichment (SELEX). The transcription product may be used in dsRNA or shRNA synthesis, for example, for RNAi technology. The transcription product may be used in CRISPR gRNA synthesis, for example, for RNP-mediated gene editing. The transcription product may be used in riboprobe synthesis, for example, for Northern blotting, or in situ hybridization. The transcription product may be used in RNA amplification. The transcription product may be used in miRNA synthesis. The transcription product may be used in anti-sense RNA synthesis.

The invention provides a method for protein expression, the method comprising introducing the linear double-stranded DNA product described herein into a prokaryotic cell or a eukaryotic cell or a cell-free protein expression system to generate a desired RNA or protein.

The invention also provides a method for production of a desired protein in vitro, comprising:
(a) providing a linear double-stranded DNA product comprising a sense strand and an antisense strand, wherein the linear double-stranded DNA product comprises one or more phosphorothioated nucleotides at internal positions in each strand; and
(b) expressing a protein from the linear double-stranded DNA product in a cell-free expression system.

The cell-free expression system may originate from a prokaryotic cell or eukaryotic cell. For example, the cell-free expression system may originate from rabbit reticulocytes, wheat germ or *Escherichia coli.*

The invention also provides a method for production of a desired protein in vivo, comprising:
(a) providing a linear double-stranded DNA product comprising a sense strand and an antisense strand, wherein the linear double-stranded DNA product comprises one or more phosphorothioated nucleotides at internal positions in each strand; and
(b) expressing a protein from the linear double-stranded DNA product in a cell.

The cell may be an animal cell, preferably a mammal cell, such as a human cell.

The desired protein might be encoded by the cassette. The invention also provides a method for production of a desired protein in vitro, comprising:
(a) providing a linear double-stranded DNA product comprising a sense strand and an antisense strand, wherein the linear double-stranded DNA product comprises a single cassette and one or more phosphorothioated nucleotides at internal positions in each strand; and
(b) expressing a protein from the cassette in a cell-free expression system.

The invention also provides a method for production of a desired protein in vivo, comprising:
(a) providing a linear double-stranded DNA product comprising a sense strand and an antisense strand, wherein the linear double-stranded DNA product comprises a single cassette and one or more phosphorothioated nucleotides at internal positions in each strand; and
(b) expressing a protein from the cassette in a cell.

The linear double-stranded DNA product may comprise at least 2, 3, 4, 5, 6, 7, 8, 9, 10 phosphorothioated nucleotides at internal positions in each strand. Preferably, the linear double-stranded DNA product comprises at least 2 phosphorothioated nucleotides at internal positions in each strand.

The invention also provides a method for production of a desired protein in vitro, comprising:
(a) providing a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
   (i)a 5'-end nucleotide of the cassette;
   (ii) a 3'-end nucleotide of the cassette; and
   (iii) one or more nucleotides outside of the cassette; and
   wherein the cassette comprises a coding sequence; and
(b) expressing a protein from the cassette in a cell-free expression system.

The invention also provides a method for production of a desired protein in vivo, comprising:
(a) providing a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
   (i)a 5'-end nucleotide of the cassette;
   (ii) a 3'-end nucleotide of the cassette; and
   (iii) one or more nucleotides outside of the cassette; and
   wherein the cassette comprises a coding sequence; and
(b) expressing a protein from the cassette in a cell.

The invention provides a method for in vivo transcription and translation of the linear double-stranded DNA product. The invention also provides a method for in vitro transcription and translation of the linear double-stranded DNA product. The linear double-stranded DNA product may have an improved or enhanced resistance to exonuclease (e.g. exonuclease III) digestion. The linear double-stranded DNA product may be resistant to exonuclease (e.g. exonuclease III) digestion. Thus, the expression (i.e. transcription and/or translation) methods described herein, which use the DNA products of enhanced resistance to exonuclease digestion, allow for a prolonged expression of the gene of interest in a cell (or in a cell-free system).

### Production of a nanoparticle or library of nanoparticles

The invention provides the use of the linear double-stranded DNA product, the complex molecule, or the library in the production of a nanoparticle or a library of nanoparticles.

### Viral vectors

Methods of producing viral vectors, such as AVV vectors, are known in the art. The most widely used method involves the co-transfection of HEK293 by three bacterial plasmids. The first plasmid encodes the *Rep* and *Cap* element, the second plasmid is a helper plasmid, while the third plasmid encodes the genetic payload of interest with inverted terminal repeats (ITRs). There are several issues surrounding the use of plasmids for viral preparation (e.g. AVV), namely: 1) the production of the plasmids is time consuming and costly, 2) there is a difficulty in the propagation of ITR sequences in *E.coli*; 3) the incorporation of the plasmid backbone into the viral capsid (e.g. AVV capsid) might be challenging (e.g. issues with antibiotic resistance markers). Together these represent the main bottleneck in viral vector production (e.g. AVV production). Thus, the invention provides a linear double-stranded DNA product suitable for use in production of viral vectors. The linear double-stranded DNA product of the invention overcomes the above issues with plasmid vectors.

The invention provides the use of the linear double-stranded DNA product, the complex molecule, or the library described herein in the production of a viral vector.

The invention provides the use of the linear double-stranded DNA product described herein in the production of a viral vector. Thus, the invention provides the use of a linear double-stranded DNA product comprising a sense strand and an antisense strand, wherein the linear double-stranded DNA product comprises a single cassette and one or more phosphorothioated nucleotides at internal positions in each strand, in the production of a viral vector. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention also provides the use of a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence, in the production of a viral vector.

The invention provides a method of producing a viral vector, the method comprising introducing the linear double-stranded DNA product, the complex molecule or the library described herein into a cell under conditions such that the viral vector is produced. The linear double-stranded DNA product, the complex molecule or the library may encode at least one element required for the production of the viral vector. For example, the linear double-stranded DNA product, the complex molecule or the library may encode Rep and/or Cap elements. The linear double-stranded DNA product, the complex molecule or the library may encode the helper plasmid elements. The linear double-stranded DNA product, the complex molecule or the library may encode Rep, Cap and helper plasmid elements. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AVV vector or lentivirus vector.

The invention also provides a method of delivering the viral vector to a cell, comprising contacting the viral vector produced by the methods described herein with the cell. The cell may be an animal cell, preferably mammal cell, such as human cell.

The invention also provides a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the present invention provides a linear double-stranded DNA product suitable for use in production of non-viral vectors. The linear double-stranded DNA product of the invention overcomes the issues of using plasmid vectors in non-viral vector preparation. For example, the linear double-stranded DNA of the invention, unlike plasmid DNA, does not comprise a bacterial backbone, allowing more transgene copies per mg of DNA. In addition, the present invention does not comprise antibiotic resistant genes and bacterial contaminants. Moreover, the linear double-stranded DNA product of the invention provides a prolonged transgene expression (due to the presence of exonuclease-resistant nucleotides), and a more cost-effective process of non-viral vector production.

The invention provides the use of the linear double-stranded DNA product described herein in the production of a non-viral vector. The invention provides the use of a linear double-stranded DNA product comprising a sense strand and an antisense strand, wherein the linear double-stranded DNA product comprises a single cassette and one or more phosphorothioated nucleotides at internal positions in each strand, in the production of a non-viral vector. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention also provides the use of a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence, in the production of a non-viral vector.

The invention also provides the use of the complex molecule described herein in the production of a non-viral vector.

The invention provides a method of delivering the non-viral vector to a cell, comprising contacting the non-viral vector comprising the linear double-stranded DNA product, the complex molecule, the library, or the composition with the cell. The cell may be an animal cell, preferably mammal cell, such as human cell.

The invention also provides a cell obtainable by the methods described herein.

### General therapeutic and diagnostic uses

The linear double-stranded DNA product, the complex molecule, the nanoparticle, the composition or the library are particularly suitable for use in therapy. These molecules may encode a sequence of a therapeutic protein, a part of a vaccine, or an element of a genetic engineering mechanism, which is used to treat a disease or infection in a subject.

The invention provides the use of the linear double-stranded DNA product, the complex molecule, the nanoparticle, the composition, or the library in therapy.

The invention provides the use of the linear double-stranded DNA product described herein in therapy. Thus, the invention provides the use of a linear double-stranded DNA product comprising a sense strand and an antisense strand, wherein the linear double-stranded DNA product comprises a single cassette and one or more phosphorothioated nucleotides at internal positions in each strand, in therapy. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention provides a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence, for use in therapy.

The invention further provides the linear double-stranded DNA product, the complex molecule, the nanoparticle, the composition, or the library for use as a medicament. The invention also provides use of the linear double-stranded DNA product, the complex molecule, the nanoparticle, the composition or the library in the manufacture of a medicament for treating a disease.

The invention further provides the linear double-stranded DNA product, the complex molecule, the nanoparticle, the composition or the library for use in treating a disease.

The invention also provides a method of treating a disease in a subject comprising administering to the subject the linear double-stranded DNA product, the complex molecule, the nanoparticle, the composition, or the library of the invention. Preferably, the amount of the linear double-stranded DNA product, the complex molecule, the nanoparticle, the composition, or the library administered to the subject is a therapeutic active amount.

The linear double-stranded DNA product, the complex molecule, the nanoparticle, the composition, or the library may be used to treat any disease or disorder. For example, the linear double-stranded DNA product, the complex molecule, the nanoparticle, the composition, or the library may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear double-stranded DNA product, the complex molecule, the nanoparticle, the composition, or the library are used to treat a genetic disorder. More preferably still, the linear double-stranded DNA product, the complex molecule, the nanoparticle, the composition, or the library are used to treat a monogenic disorder. For example, the linear double-stranded DNA product, the complex molecule, the nanoparticle, the composition, or the library may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

A subject treated with the linear double-stranded DNA product, the complex molecule, the nanoparticle, the composition, or the library may receive the linear double-stranded DNA product, the complex molecule, the nanoparticle, or the library in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

The linear double-stranded DNA product, the complex molecule, the nanoparticle, the composition, or the library is preferably administered to the subject as a composition or a pharmaceutical composition as described herein.

As used herein, "administering" means introducing the linear double-stranded DNA product, the complex molecule, the nanoparticle, the composition, or the library into the subject's body as described in more detail above (see "Compositions"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the linear double-stranded DNA product, the complex molecule, the nanoparticle, the composition, or the library that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides use of the linear double-stranded DNA product, the complex molecule, the nanoparticle, the composition, or the library in a method of diagnosing a disease and/or a disorder.

The invention provides the use of the linear double-stranded DNA product, the complex molecule, the nanoparticle, the composition, or the library in the "in vitro" diagnosis of a disease.

The invention also provides the linear double-stranded DNA product, the complex molecule, the nanoparticle, the composition, or the library for use in a method of diagnosis "in vivo" of disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear double-stranded DNA product, the complex molecule, the nanoparticle, the composition, or the library are used to diagnose a genetic disorder. More preferably still, the linear double-stranded DNA product, the complex molecule, the nanoparticle, the composition, or the library are used to diagnose a monogenic disorder. For example, the linear double-stranded DNA product, the complex molecule, the nanoparticle, the composition, or the library may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the linear double-stranded DNA product, the complex molecule, the nanoparticle, the composition, or the library.

To facilitate detection and/or quantification of the linear double-stranded DNA product, the linear double-stranded DNA product may be attached or bound to a functional portion. The functional portion may be any functional portion described herein. For example the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the DNA product. For example, the signal may be measured by flow cytometry and/or fluorescence-activated cell sorting of a linear double-stranded DNA product attached to a fluorescent probe.

The linear double-stranded DNA product may be detected by being bound to a capture moiety, for example in a lateral flow assay. In this example, the functional portion is a protein, for example, an antibody specific for the capture moiety. The capture of the antibody attached to the linear double-stranded DNA product may produce a visual signal (e.g. a band of a different colour).

The invention also provides a method that combines diagnosis of a disease with a treatment of the disease.

### Cell therapy

As discussed above, the products of the invention may be substantially less contaminated than plasmid DNA. In addition, the products of the invention are very simple in nature (e.g. no bacterial backbone), which means that they are typically easy to work with. Since, at minimum, the products of the invention comprise the gene of interest, more copies of the gene of interest may be obtained per reaction volume (or per mass of the final product). The pure and simple nature of the products described herein makes them particularly suitable for use in cell therapy. For example, a cell comprising the linear double-stranded DNA product, the complex molecule, the nanoparticle, the composition and the library described herein may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the linear double-stranded DNA product, the complex molecule, the nanoparticle, the composition, or the library for use in cell therapy.

The invention provides the linear double-stranded DNA product described herein for use in cell therapy. Thus, the invention provides a linear double-stranded DNA product comprising a sense strand and an antisense strand, wherein the linear double-stranded DNA product comprises a single cassette and one or more phosphorothioated nucleotides at internal positions in each strand, for use in cell therapy. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention also provides a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence, for use in cell therapy.

The invention also provides the use of the linear double-stranded DNA product, the complex molecule, the nanoparticle, the composition, or the library in the production of cell therapy. The invention provides a method of cell therapy, comprising contacting the linear double-stranded DNA product, the complex molecule, the nanoparticle, the library, the viral or non-viral vector, or the composition with a cell. Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

### Vaccines

The products of the invention are particularly suitable for use in vaccine production. The vaccine may comprise the linear double-stranded DNA product, the complex molecule or the library described herein. Alternatively, the linear double-stranded DNA product, the complex molecule, or the library described herein may be used to produce a vaccine, preferably an mRNA-based vaccine. For example, vaccines such as the BioNTech and Moderna mRNA vaccines against COVID-19.

Thus, the invention provides the use of the linear double-stranded DNA product, the complex molecule, the nanoparticle, the composition, or the library described herein in the production of a vaccine.

The invention provides the use of the linear double-stranded DNA product described herein in the production of a vaccine. The invention provides the use of a linear double-stranded DNA product comprising a sense strand and an antisense strand, wherein the linear double-stranded DNA product comprises a single cassette and one or more protected nucleotides (e.g. phosphorothioated nucleotides) at internal positions in each strand, in the production of a vaccine. Preferably, the one or more protected nucleotides (e.g. phosphorothioated nucleotides) at the internal positions in each strand may be selected from:
(a) a 5'-end nucleotide of the cassette;
(b) a 3'-end nucleotide of the cassette; and
(c) one or more nucleotides outside of the cassette.

The invention also provides the use of a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence, in the production of a vaccine.

Preferably, the vaccine is an mRNA vaccine.

The invention also provides a vaccine comprising a linear double-stranded DNA product comprising a sense strand and an antisense strand, wherein the linear double-stranded DNA product comprises a single cassette and one or more protected nucleotides (e.g. phosphorothioated nucleotides) at internal positions in each strand. Preferably, the one or more protected nucleotides (e.g. phosphorothioated nucleotides) at the internal positions in each strand may be selected from:
(a) a 5'-end nucleotide of the cassette;
(b) a 3'-end nucleotide of the cassette; and
(c) one or more nucleotides outside of the cassette.

The invention provides a vaccine comprising a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(d) a 5'-end nucleotide of the cassette;
(e) a 3'-end nucleotide of the cassette; and
(f) one or more nucleotides outside of the cassette; and
wherein the cassette comprises a coding sequence.

The linear double-stranded DNA product may encode an antigen, which may cause an immune response in a subject. The subject may be human. Preferably, the antigen is encoded on the cassette.

### CAR-T cells

The invention provides the use of the linear double-stranded DNA product, the complex molecule, the nanoparticle, the composition, or the library described herein in the production of a CAR-T cell.

The invention provides the use of the linear double-stranded DNA product described herein in the production of a CAR-T cell. The invention provides the use of a linear double-stranded DNA product comprising a sense strand and an antisense strand, wherein the linear double-stranded DNA product comprises a single cassette and one or more phosphorothioated nucleotides at internal positions in each strand, in the production of a CAR-T cell. Preferably, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette.

The invention also provides the use of a linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence, in the production of a CAR-T cell.

The invention provides a method for production of a genetically engineered CAR-T cell comprising: (a) introducing the linear double-stranded DNA product, the complex molecule, the nanoparticle, the composition, or the library described herein into a T cell; and (b) expressing a gene of interest. Preferably, the gene of interest is a tumour-specific CAR.

The invention provides a method for production of a genetically engineered CAR-T cell comprising: (a) introducing a linear double-stranded DNA product comprising a sense strand and an antisense strand, wherein the linear double-stranded DNA product comprises a single cassette and one or more phosphorothioated nucleotides at internal positions in each strand into a T cell; and (b) expressing a gene of interest. Preferably, the gene of interest is a tumour-specific CAR.

The method may further comprise, before step (a), a step of removing mononuclear cells from a patient. Preferably, the step of removing is performed using leukapheresis. Preferably the mononuclear cells are T cells. The method may further comprise, after step (b), a step of returning the genetically engineered cells to the patient.

The invention also provides an engineered T cell obtainable by any methods described herein. The engineered T cell may be suitable for use in CAR T-cell therapy.

### CRISPR delivery

The products of the invention are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles). As discussed above, the present invention provides a linear double-stranded DNA product that has low dispersity. Thus, the nanoparticles (or library of nanoparticles), or viral or non-viral delivery vectors comprising the linear double-stranded DNA are less disperse (i.e. more uniform), which improves the efficiency of cell delivery.

The linear double-stranded DNA product may comprise a gene sequence encoding any component of the CRIPSR machinery. The linear double-stranded DNA product may encode all components of the CRIPSR machinery.

The linear double-stranded DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The linear double-stranded DNA product encoding the repair template may be delivered to a cell by a nanoparticle, a non-viral vector or a viral vector, or without the aid of any carrier.

The linear double-stranded DNA product may comprise a gene sequence encoding a nuclease protein of a CRISPR system (e.g. Cas9, Cpf1, or MAD7), and/or a guide RNA. The linear double-stranded DNA product may comprise a gene sequence encoding a nuclease protein of a CRISPR system (e.g. Cas9, Cpf1, or MAD7). The linear double-stranded DNA product may comprise a gene sequence encoding a guide RNA. The linear double-stranded DNA product may comprise a gene sequence encoding a nuclease protein of a CRISPR system (e.g. Cas9, Cpf1, or MAD7) and a guide RNA. The linear double-stranded DNA product may comprise a gene sequence encoding a genomic target to be modified (e.g. a spacer). The linear double-stranded DNA product may be ligated to a vector. The vector may comprise a sequence of some of the components of the CRIPSR system. The vector may comprise a sequence of guide RNA or a sequence of a part of the guide RNA. If the vector comprises a sequence of a part of the guide RNA, the linear double-stranded DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The linear double-stranded DNA product may encode the nuclease of the CRISPR system and guide RNA. One linear double-stranded DNA product may encode the nuclease of the CRISPR system, and the other linear double-stranded DNA product may encode guide RNA.

The linear double-stranded DNA product may be for use in CRISPR-Cas mediated repair by recombination, homology-directed repair, or non-homologous end joining.

If the nuclease of the CRISPR system and the guide RNA are encoded by a different linear double-stranded DNA product, they may be part of a different or the same delivery mechanism. For example, the linear double-stranded DNA product encoding the nuclease of the CRISPR system may be delivered to a cell by a first nanoparticle, non-viral vector or viral vector, whereas the linear double-stranded DNA product encoding the guide RNA may be delivered to a cell by a second nanoparticle, non-viral vector, or viral vector. For example, the linear double-stranded DNA product encoding the nuclease of the CRISPR system and the linear double-stranded DNA product encoding the guide RNA may be delivered to a cell by the same nanoparticle, non-viral vector or viral vector.

If the nuclease of the CRISPR system and the guide RNA are encoded by the same linear double-stranded DNA product (or by a vector that comprises the linear double-stranded DNA product) they may be part of the same delivery mechanism. For example, the linear double-stranded DNA product (or the vector) encoding the nuclease of the CRISPR system and the guide RNA may be delivered to a cell by a nanoparticle, a non-viral vector or a viral vector.

Thus, the invention provides the linear double-stranded DNA product, the complex molecule, the composition, the library, or the nanoparticle for use in CRISPR system delivery to a cell. The invention provides a method of delivering the CRISPR system to a cell, comprising contacting the linear double-stranded DNA product, the complex molecule, the nanoparticle, the library, or the composition with a cell.

The cell may be an animal cell, preferably mammal cell, such as human cell.

The invention also provides a cell obtainable by the methods described herein. The cell is particularly suitable for use in cell therapy and/or in vivo therapy.

The linear double-stranded DNA product, the complex molecule, the nanoparticle or the library of the invention may be used in transcription, to generate RNA, preferably mRNA, in vitro or in vivo.

### 8. Methods of producing linear double-stranded DNA products and libraries thereof

The double-stranded linear DNA product or library thereof may be a product of any amplification process. The amplification process may be an *in vitro* or *in vivo* amplification process. Preferably, the amplification process is an *in vitro* amplification process. For example, the amplification process may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification process is performed by rolling circle amplification. Preferably, the double-stranded DNA product is a product of rolling circle amplification. The double-stranded DNA product may be generated by a linear RCA, an exponential RCA, or a multiply-primer RCA. Rolling circle amplification may be performed without any primers, or in the presence of a primer or multiple primers. For example, the primer may be a synthetic primer. The primers may be random primers. Rolling circle amplification may be performed in the presence of a primase. The primase may be *Tth*PrimPol. Preferably, if the rolling circle amplification is performed without any primers, it is performed in the presence of a primase, such as *Tth*PrimPol. Similarly, if a primer is used during amplification reaction, a primase is not used. The double-stranded DNA product may be generated by the rolling circle amplification *in vitro* under isothermal conditions using a suitable nucleic acid polymerase, such as Phi29 DNA polymerase. Rolling circle amplification may be performed in the presence of protected nucleotides, such as phosphorothioated nucleotides.

The inventors of the present application has surprisingly discovered a method of cleaving the product of rolling circle amplification so that only a single copy of the cassette (which may comprise a gene of interest) is present in a single linear double-stranded DNA product. A single copy of the cassette per single DNA product provides many benefits over known amplification products. For example, a single copy of the cassette per single DNA product ensures that the DNA product is as small as possible, which play a key role in, for example, effectiveness of a cell delivery method and/or cell delivery system.

The linear double-stranded DNA product described herein or library thereof may be produced by cleavage of the amplification product. The cleavage may be enzymatic cleavage, for example, endonuclease digestion, such as restriction enzyme digestion. After cleavage, the linear double-stranded DNA product may comprise a single cassette (e.g. a cassette comprising a single coding sequence). That is to say, after cleavage, the linear double-stranded DNA product may not comprise or consist of a plurality of tandem repeat sequences, or concatemeric DNA. After cleavage, the linear double-stranded DNA product may not comprise or consist of the concatemeric DNA which is produced by known methods, for example as described in US Patent US10350307 B2. After cleavage, the linear double-stranded DNA product may comprise a single copy of the DNA sequence of interest, for example, a single copy of the coding sequence.

Thus, the invention provides a method for production of the linear double-stranded DNA product or the library of linear double-stranded DNA products described herein, comprising:
(a) amplification of a DNA template molecule comprising at least one cleavable sequence in the presence of at least one type of a protected nucleotide to generate a double-stranded DNA product; and
(b) contacting the double-stranded DNA product with at least one enzyme to generate the linear double-stranded DNA product or library thereof, preferably wherein the linear double-stranded DNA product has increased resistance to nucleases.

The amplification method may employ a rolling circle amplification method. Thus, the method of production of the linear double-stranded DNA product or the library of linear double-stranded DNA products described herein may comprise:
(a) rolling circle amplification of a DNA template molecule comprising at least one cleavable sequence in the presence of at least one type of a protected nucleotide to generate a double-stranded DNA product; and
(b) contacting the double-stranded DNA product with at least one enzyme to generate the linear double-stranded DNA product or library thereof, preferably wherein the linear double-stranded DNA product has increased resistance to nucleases.

The cleavable sequence may be a sequence that is cleaved by an enzyme. Preferably, the cleavable sequence is a sequence that is cleaved by an endonuclease, such as restriction endonuclease. The method of production of the linear double-stranded DNA product or the library of linear double-stranded DNA products described herein may comprise:
(a) rolling circle amplification of a DNA template molecule comprising at least one endonuclease target sequence in the presence of at least one type of a protected nucleotide to generate a double-stranded DNA product; and
(b) contacting the double-stranded DNA product with at least one enzyme to generate the linear double-stranded DNA product or library thereof, preferably wherein the linear double-stranded DNA product has increased resistance to nucleases.

Preferably, the linear double-stranded DNA product is resistant to exonuclease digestion, such as exonuclease III digestion. Thus, the method may use nucleotides that provide protection against exonuclease digestion, such as phosphorothioated nucleotides or MOE nucleotides. Accordingly, the invention provides a method for production of the linear double-stranded DNA product or the library of linear double-stranded DNA products described herein, comprising:
(a) rolling circle amplification of a DNA template molecule comprising at least one endonuclease target sequence in the presence of at least one type of a phosphorothioated nucleotide to generate a double-stranded DNA product; and
(b) contacting the double-stranded DNA product with at least one endonuclease to generate the linear double-stranded DNA product, wherein the linear double-stranded DNA product has increased resistance to exonucleases.

As described herein, the linear-double-stranded DNA product may comprise a cassette. Thus, a method for in vitro production of a linear double-stranded DNA product, or library of linear double-stranded DNA products, having increased resistance to exonucleases may comprise the steps of:
(a) rolling circle amplification of a DNA template molecule comprising at least one endonuclease target sequence in the presence of at least one type of a phosphorothioated nucleotide to generate a double-stranded DNA product,
   wherein the double-stranded DNA product comprises a single cassette; and
(b) contacting the double-stranded DNA product with at least one endonuclease to generate a linear double-stranded DNA product comprising a single cassette, or library of such double-stranded DNA products, preferably wherein the linear double-stranded DNA product has increased resistance to exonucleases.

The ratio of phosphorothioated nucleotides to total nucleotides used in the methods described herein may be at least 0.0001, at least 0.0025, at least 0.025, at least 0.10, at least 0.12 at least 0.15, at least 0.25, at least 0.35, at least 0.5, or at least 0.75. The ratio of the phosphorothioated nucleotides to total nucleotides used in the methods described herein may be less than 1, less than 0.9, less than, 0.8, less than 0.65, less than 0.5, less than 0.4, less than 0.3, less than 0.2, or less than 0.1. The ratio of the phosphorothioated nucleotides to total nucleotides may be between 0.0001 -1, between 0.0025-0.75, between 0.025-0.65, between 0.025-0.15, or between 0.25-0.50. Preferably, a ratio of the phosphorothioated nucleotides to total nucleotides is between 0.025-0.15. Preferably, a ratio of the phosphorothioated nucleotides to total nucleotides is about 0.025, about 0.10, about 0.12, about 0.15, or about 0.25. More preferably still, a ratio of the phosphorothioated nucleotides to total nucleotides is about 0.025. As shown in the Examples, a ratio of the phosphorothioated nucleotides to total nucleotides of about 0.025 is sufficient to provide enhanced resistance to exonuclease digestion.

The method for production of the linear double-stranded DNA product or library of double-stranded DNA products described herein may comprise:
(a) rolling circle amplification of a DNA template molecule comprising at least one endonuclease target sequence in the presence of at least one type of a MOE nucleotide to generate a double-stranded DNA product; and
(b) contacting the double-stranded DNA product with at least one endonuclease to generate the linear double-stranded DNA product or library thereof, wherein the linear double-stranded DNA product has increased resistance to exonucleases.

Although at least one type of the protected nucleotide is sufficient to provide protection against exonuclease digestion, the method for production of the linear double-stranded DNA product described herein may use at least two, at least three or at least four different protected nucleotides, such as phosphorothioated nucleotides.

In the methods described herein, the DNA template molecule may comprise at least one cleavable sequence. The cleavable sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. For example, the restriction endonuclease target sequence may be a Bsal, Ndel, Smal, Nsil and/or Xbal target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the linear double-stranded DNA product.

Thus, the method for production of the linear double-stranded DNA product or the library of linear double-stranded DNA products described herein may comprise:
(a) introducing at least one cleavable sequence (e.g. restriction endonuclease target sequence) to a DNA template molecule;
(b) amplification (e.g. rolling circle amplification) of the DNA template molecule comprising the at least one cleavable sequence in the presence of at least one type of a protected nucleotide (e.g. phosphorothioated nucleotide) to generate a double-stranded DNA product; and
(c) contacting the double-stranded DNA product with at least one enzyme (e.g. restriction endonuclease) to generate the linear double-stranded DNA product or library thereof, preferably wherein the linear double-stranded DNA product has increased resistance to nucleases (e.g. exonuclease III).

The inventors of the present application have surprising discovered that the enzyme capable of cleaving the cleavable sequence is much less efficient at cleaving if at least one protected nucleotide is incorporated into the cleavable sequence of the double-stranded DNA product. In other words, to accomplish substantially full digestion of the cleavable sequence (i.e. release of substantially all linear double-stranded DNA product), the at least one type of protected nucleotide may not be incorporated into the cleavable sequence of the double-stranded DNA product. Thus, the at least one type of protected nucleotide (e.g. phosphorothioated nucleotide) used in the method may be selected so that it does not form a part of the cleavable sequence. For example, the Smal restriction enzyme recognises the cleavable sequence: 5'-CCCGGG-3' (and complementary 3'-GGGCCC-5'), thus, preferably the at least one type of protected nucleotide used in the method may be, for example, α-S-dATP. The use of only α-S-dATP as the protected nucleotide in this example would ensure that the cleavable sequence is free of the protected nucleotides. However, some types of cleavable sequences may comprise at least 4 types of nucleotides. For example, Bsal restriction enzyme recognises 5'-GGTCTC(N)-3' and complementary 3'-CCAGAG(N)-5'. The inventors of the present application have further discovered that any type of protected nucleotide may be used, as long as the ratio of protected nucleotides to total nucleotides is less than 0.5. Preferably, the ratio of the protected (e.g. phosphorothioated) nucleotides to total nucleotides used is less than 0.5, 0.4, 0.3, 0.2, or 0.1, more preferably still, the ratio is less than 0.3.

The step of generation of the linear double-stranded DNA product or library thereof may release at least 80%, 85%, 90%, 95%, or 100% of the linear double-stranded DNA product or library thereof. Preferably, the step of generation of the linear double-stranded DNA product or library thereof releases at least 90% of the linear double-stranded DNA product or library thereof.

The released linear double-stranded DNA product preferably comprises a single cassette and/or a single gene of interest. Thus, the method may comprise the steps of:
(a) introducing at least one cleavable sequence (e.g. restriction endonuclease target sequence) to a DNA template molecule;
(b) rolling circle amplification of the DNA template molecule comprising the at least one cleavable sequence (e.g. restriction endonuclease target sequence) in the presence of at least one type of a phosphorothioated nucleotide to generate a double-stranded DNA product,
   wherein the double-stranded DNA product comprises a single cassette; and
(c) contacting the double-stranded DNA product with at least one enzyme (e.g. restriction endonuclease) to generate a linear double-stranded DNA product comprising the single cassette, or library of such linear double-stranded DNA products.

The step of generation of the linear double-stranded DNA product comprising the single cassette may release at least 80%, 85%, 90%, 95%, or 100% of the linear double-stranded DNA product. Preferably, the step of generation of the linear double-stranded DNA product releases at least 90% of the linear double-stranded DNA product. Preferably, the released linear double-stranded DNA product comprises a single cassette (or gene of interest).

After cleavage, the library of linear double-stranded DNA product may have low dispersity (i.e. be substantially monodisperse). As used herein in the context of a linear double-stranded DNA product, the terms "low dispersity" and "monodisperse" are intended to encompass a collection of copies of linear double-stranded DNA product of substantially the same size, i.e. the same length of the polynucleotide chain. That is to say, each linear double-stranded DNA product in the library may vary in size (i.e. number of bases in the polynucleotide chain) from the other linear double-stranded DNA products in the library by less than 10%, preferably by less than 5%. This is in contrast to a library of polydisperse linear double-stranded DNA products, which refers to a collection of copies of the DNA product that have an inconsistent size, i.e. an inconsistent length of the polynucleotide chain.

The inventors of the present application have discovered a method of production of a substantially monodisperse library of linear double-stranded DNA products which is preferably resistant to exonuclease digestion. The substantially monodisperse nature of the library of linear double-stranded DNA products described herein provides a benefit over known DNA products in that it is suitable for nanoparticle and viral or non-viral vector production. The produced nanoparticles, viral, and/or non-viral vectors will have a substantially uniform size which facilitates transformation and/or transfection and play a key role in internalization and drug release processes.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, or (iv) a bacterial artificial chromosome (BAC). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be at least 70%, 80, 90%, 95%, 96%, 97%, 98%, 99%, or 100% complementary with the linear double-stranded DNA product. Preferably, the DNA template is at least 99% complementary with the linear double-stranded DNA product.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear double-stranded DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

In the methods described herein, the type of protected nucleotide (e.g. phosphorothioated nucleotide) used may be complementary or identical to at least one nucleotide of the homopolymeric sequence of the DNA template molecule. Preferably, the protected nucleotide is complementary to the nucleotides of the homopolymeric sequence. For example, if the homopolymeric sequence is a polyA sequence, the phosphorothioated nucleotide used in the method, and, preferably, incorporated into the double-stranded DNA product may be α-S-dTTP. If the homopolymeric sequence is a polyC sequence, the phosphorothioated nucleotide used in the method, and, preferably, incorporated into the double-stranded DNA product may α-S-dGTP. If the homopolymeric sequence is a polyG sequence, the phosphorothioated nucleotide used in the method, and, preferably, incorporated into the double-stranded DNA product may be α-S-dCTP. If the homopolymeric sequence is a polyT sequence, the phosphorothioated nucleotide used in the method, and, preferably, incorporated into the double-stranded DNA product may be α-S-dATP. The methods described herein may use different types of protected nucleotides in a single reaction. Thus, for example, although the homopolymeric sequence is a polyA sequence, at least two, three, or four different types of protected nucleotides could be used in the method. Preferably, all four of α-S-dATP, α-S-dCTP, α-S-dGTP, and α-S-dTTP are used in the method.

The DNA template may comprise two different homopolymeric sequences. For example, before the DNA template is circularized, the DNA template, may comprise, at each end of the strand, a different homopolymeric sequence. For example, the 3'-end of the DNA template may comprise a polyA sequence and the 5'-end of the DNA template may comprise a polyT sequence.

As described herein, the linear double-stranded DNA product generated by the methods described herein may comprise a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette.

A linear double-stranded DNA product may be obtainable by any of the methods provided herein.

The invention provides a method for producing a linear double-stranded DNA product with protected ends, or a library of such linear double-stranded DNA products. The method may comprise the steps of:
(a) addition of at least one homopolymeric sequence to a DNA template molecule
(b) amplification of a DNA template molecule comprising at least one endonuclease target sequence and at the least one homopolymeric sequence in the presence of at least one type of a protected nucleotide to generate a double-stranded DNA product,
(c) contacting the double-stranded DNA product with at least one endonuclease to generate the linear double-stranded DNA product, and
(d) contacting the linear double-stranded DNA product with exonuclease to generate a linear double-stranded DNA product with protected ends, or a library of such linear double-stranded DNA products.

Any DNA template molecules described herein are suitable for use in the method for producing a linear double-stranded DNA product with protected ends.

The addition of at least one homopolymeric sequence to the DNA template molecule may take place before the DNA template molecule is circularized. Preferably, two homopolymeric sequences are added to the DNA template molecule. The two homopolymeric sequences may be different or the same. The homopolymeric sequence may be added to a 5'-end or a 3'-end or both a 5'-end and a 3'-end of the DNA template molecule. The homopolymeric sequence may be a polyA, a polyC, a polyG, or a polyT sequence. For example, before the DNA template is circularized, two different homopolymeric sequences may be added to the DNA template molecule. For example, a polyC sequence may be added to the 3'-end of the DNA template and a polyG sequence may be added to the 5'-end of the DNA template molecule.

The homopolymeric sequence may be between 3-200 nucleotides in length. The homopolymeric sequence may be used to facilitate purification of the linear double-stranded DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The step of addition of at least one homopolymeric sequence to the DNA template molecule may be performed by any conventional methods known in the art. For example, the homopolymeric sequence may be ligated to the DNA template molecule using a DNA ligase enzyme. The homopolymeric sequence may be attached to the DNA template molecule using restriction enzyme approach. The homopolymeric sequence may attached to the DNA template molecule using any synthetic biologic methods, for example successive hybridization assembling (SHA).

Preferably, amplification is rolling circle amplification. The rolling circle amplification typically requires circular template molecules. Thus, after the step of addition of at least one homopolymeric sequence to a DNA template molecule, the method may further comprise a step of circularization of the DNA template molecule. Circularization may be performed by any methods known in the art. For example, a circular DNA template molecule may be obtained from recombinase reaction, preferably Cre recombinase reaction, or from a ligase reaction, preferably using the golden gate assembly. The circular DNA template molecule may be obtained by covalently-closing linear DNA molecule. For example, the DNA template molecule may be a DNA molecule processed with TelN protelomerase, or a DNA molecule generated by ligation of the DNA ends with an adaptor.

The step of contacting the linear double-stranded DNA product with exonuclease to generate a linear double-stranded DNA product with protected ends may use exonuclease III. Exonucleases cleave unprotected nucleotides from either the 5'-end or the 3'-end of the polynucleotide chain. Thus, exonuclease may digest the linear double-stranded DNA product up to the point of encountering a protected nucleotide in the strand (e.g. a phosphorothioated nucleotide). The exonuclease may be removed from the reaction once the ends of the linear double-stranded DNA product are protected from further exonuclease digestion, preferably exonuclease III digestion. Thus, the method may comprise a further step of removing the exonuclease.

Preferably, the linear double-stranded DNA product further comprises one or more protected nucleotides at an internal positions in at least one strand, preferably in both strands.

The at least one type of protected nucleotide (e.g. phosphorothioated nucleotide) used may be complementary or identical to at least one nucleotide of the homopolymeric sequence of the DNA template molecule. Preferably, the protected nucleotide is complementary to the nucleotides of the homopolymeric sequence. Preferably, the protected nucleotide is phosphorothioated nucleotide. For example, if the homopolymeric sequence is a polyA sequence, the phosphorothioated nucleotide used in the method, and, preferably, incorporated into the double-stranded DNA product may be α-S-dTTP. If the homopolymeric sequence is a polyC sequence, the phosphorothioated nucleotide used in the method, and, preferably, incorporated into the double-stranded DNA product may α-S-dGTP. If the homopolymeric sequence is a polyG sequence, the phosphorothioated nucleotide used in the method, and, preferably, incorporated into the double-stranded DNA product may be α-S-dCTP. If the homopolymeric sequence is a polyT sequence, the phosphorothioated nucleotide used in the method, and, preferably, incorporated into the double-stranded DNA product may be α-S-dATP. The method may use different types of protected nucleotides in a single reaction. Thus, for example, although the homopolymeric sequence is a polyA sequence, at least two, three, or four different types of protected nucleotides may be used in the method. Preferably, all four of α-S-dATP, α-S-dCTP, α-S-dGTP, and α-S-dTTP are used in the method.

The addition of the homopolymeric sequence to the template DNA molecule ensures that one protected nucleotides will be added to the complementary strand of the DNA product. For example, if a polyT sequence is incorporated into the template DNA molecule, and all dATPs are substituted with protected α-S-dATP and used during amplification, the resulting product will comprise a protected poly-α-S-dATP end. Similarly, if a polyA sequence of 8 nucleotides is incorporated into the template DNA molecule, and 25% of all dTTPs are substituted with protected α-S-dTTP and used during amplification, the resulting product will comprise, on average, two α-S-dTTP in the end region of the DNA product.

The protection of the 5'-end of the DNA product may be accomplished by any methods described herein. Similarly, protection of the 3'-end of the DNA product may be accomplished by any methods described herein. However, a method for producing a linear double-stranded DNA product with protected 3'-ends does not need to rely on the incorporation of a homopolymeric sequence to the template DNA molecule. For example, amplification of the DNA template molecule may be carried out in the absence of protected nucleotides to generate a double-stranded DNA product. The double-stranded DNA product may be digested to produce single units of the DNA cassette of interest. The DNA cassettes may be enzymatically modified to incorporate protected nucleotides at the 3' end.

Thus, in one example, the method may comprise: (1) digestion of the double-stranded DNA product with a restriction enzyme generating 3' recessive ends, and (2) incorporation of protected nucleotides into the 3' recessive ends by a DNA polymerase (e.g.T4 DNA polymerase, or DNA Polymerase I large (Klenow) fragment) to produce a double-stranded DNA product with protected 3'-ends.

In another example, the method may comprise: (1) digestion of the double-stranded DNA product with a restriction enzyme generating blunt ends, and (2) extension of the 3' blunt ends by Terminal Transferase (TdT) using protected nucleotides.

The method for producing a linear double-stranded DNA product with protected ends, or a library of linear double-stranded DNA products, may rely on the combination of methods described herein. For example, the protection at 5'-end may be accomplished by using homopolymeric sequences in the template molecule, while protection at the 3'-end may be accomplished by incorporation of protected nucleotides into the 3' recessive ends by a DNA polymerase (after restriction enzyme digestion).

Conventional cell-based methods for production of linear DNA are costly and have a considerable manufacturing footprint, requiring the use of large volumes of bacteria cultures grown under sterile conditions in fermenters. Obtaining the amplified DNA from the bacteria requires cell lysis, releasing endotoxins which are toxic to mammalian cells. As such, the DNA product must be purified to remove these bacterial contaminants, particularly for use as a therapeutic agent. The complex biochemical milieu of the host bacterial cell can lead to reproducibility issues between batches, with varying yield and quality of DNA. Thus, the invention provides an improved method of manufacturing a linear double-stranded DNA product which has improved resistance to nuclease digestion (e.g. exonuclease digestion). The described manufacturing process may be cell-free, making the process less time consuming and more economical.

### BRIEF DESCRIPTION OF THE DRAWINGS

The invention, together with further objects and advantages thereof, may best be understood by making reference to the description taken together with the accompanying drawings, in which:

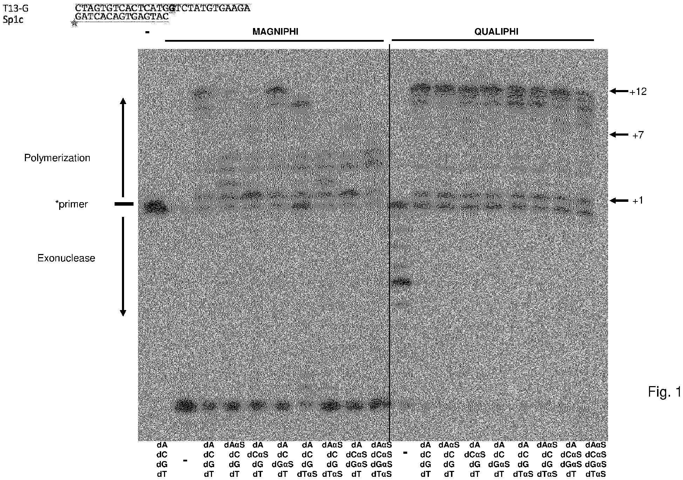

Figure 1 illustrates Phi29DNApol polymerization activity in the presence of different combinations of 2'-deoxynucleotides-5'-triphosphate (dNTP) and phosphorothioated nucleotides (2'-deoxynucleotides-5'-(α-thio)-triphosphate; or α-S-dNTP). The two forms of Phi29DNApol tested (wild-type and QualiPhi chimera) extended with different efficiencies a primer molecule (SEQ ID NO: 2) according to the offered template (SEQ ID NO: 3) by polymerizing deoxynucleotides. Conditions: 2.5 nM DNA, 30 nM Phi29DNApol, 10 mM MgCl₂, 50 nM dNTPs, 5 min at 30°C.

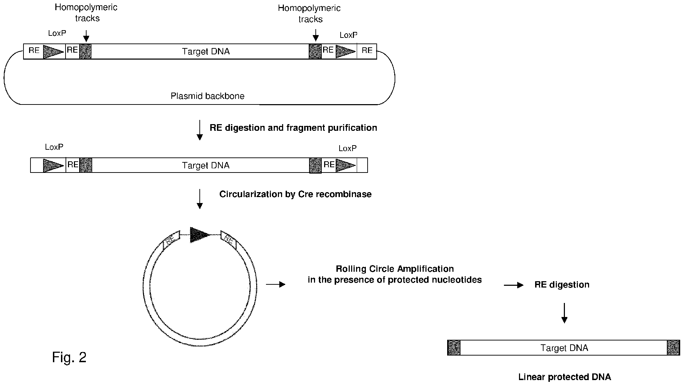

Figure 2 illustrates an exemplary method of production of the linear double-stranded DNA product using rolling circle amplification. The workflow to obtain protected linear DNA molecules by the addition of 2'-deoxynucleotides-5'-(α-thio)-triphosphate during rolling circle amplification (RCA) of a circular DNA template generated through the action of Cre recombinase on substrates containing two LoxP sequences (SEQ ID NO: 4) flanking the DNA of interest in the same direction. Cre reaction conditions: reaction volume 50 µl, DNA of interest purified from agarose gel electrophoresis after restriction enzyme digestion (100 ng), Cre recombinase (NEB, 4 units), incubation time and temperature: 30 min at 37°C and 20 min at 80°C. Next, to remove remaining non-circular DNA molecules before the amplification step, *E. coli* exonuclease I (NEB, 20 units) and III (NEB, 100 units) were added and the reaction was incubated 30 min at 37°C and 20 min at 80°C. Before the amplification, circularized DNA samples were first denatured by adding 2.5 µl of buffer D (400 mM KOH, 10 mM EDTA) and incubating 3 min at room temperature. Samples were then neutralized by adding 2.5 µl of buffer N (400 mM HCI, 600 mM Tris-HCI pH 7.5). Rolling circle amplification conditions: 50 µl reaction volume, 5 µl TruePrime WGA reaction buffer 10x (4basebio), 7.5 µl denatured DNA sample, 5 µl *Tth*PrimPol (1 µM) or 5 µl random synthetic hexamers (500 µM), 0.8 µl QualiPhi Phi29DNApol (12,5 µM), 2.5 units PPase (Thermo), 5 µl dNTPs (10 mM) with the indicated proportion of 2'-deoxynucleotides-5'-(α-thio)-triphosphate in each case. Incubation time and temperature: 20 hours at 30°C and 10 min at 65°C.

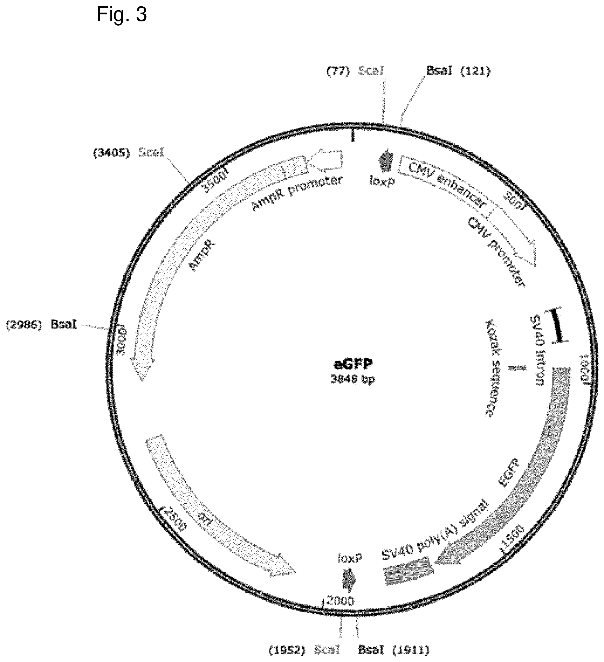

Figure 3 illustrates an exemplary template DNA molecule used in the methods described herein. A mammalian expression cassette is formed by CMV promoter (SEQ ID NO: 5) and enhancer (SEQ ID NO: 6), eGFP reporter gene (SEQ ID NO: 7) and SV40 polyA signal (SEQ ID NO: 8). The expression cassette is flanked by two LoxP sequences (SEQ ID NO: 4) having the same orientation.

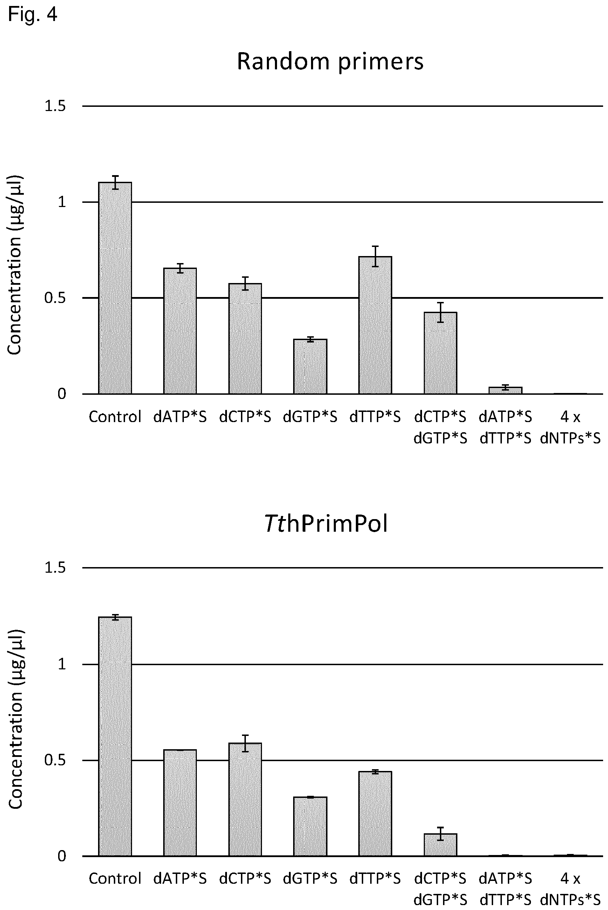

Figure 4 illustrates amplification yields in the presence and absence of phosphorothioated nucleotides. Specifically, the Figure illustrates Picogreen quantification of the amplification yields obtained from Cre-derived circular DNA molecules substituting unprotected 2'-deoxynucleotides-5'-triphosphate (dNTP) with 2'-deoxynucleotides-5'-(α-thio)-triphosphate (dNTP*S). Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP or dNTP*S, incubation time and temperature: 20 hours at 30°C and 10 min at 65°C.

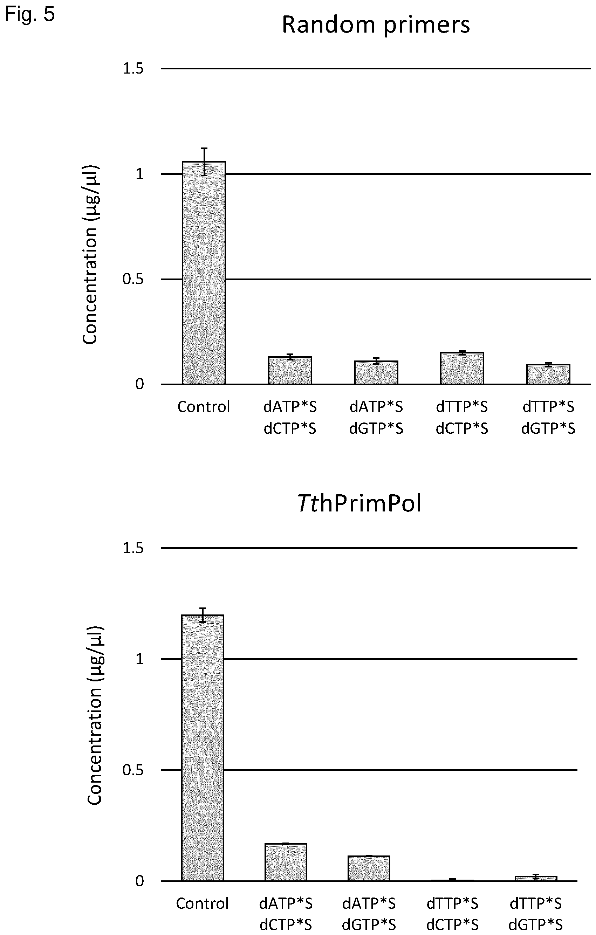

Figure 5 illustrates amplification yields in the presence and absence of phosphorothioated nucleotides. Specifically, this Figure illustrates Picogreen quantification of the amplification yields obtained from Cre-derived circular DNA molecules substituting non-complementary pairs of natural unprotected 2'-deoxynucleotides-5'-triphosphate (dNTP) for their phosphorothioated counterparts. Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP or dNTP*S, incubation time and temperature: 20 hours at 30°C and 10 min at 65°C.

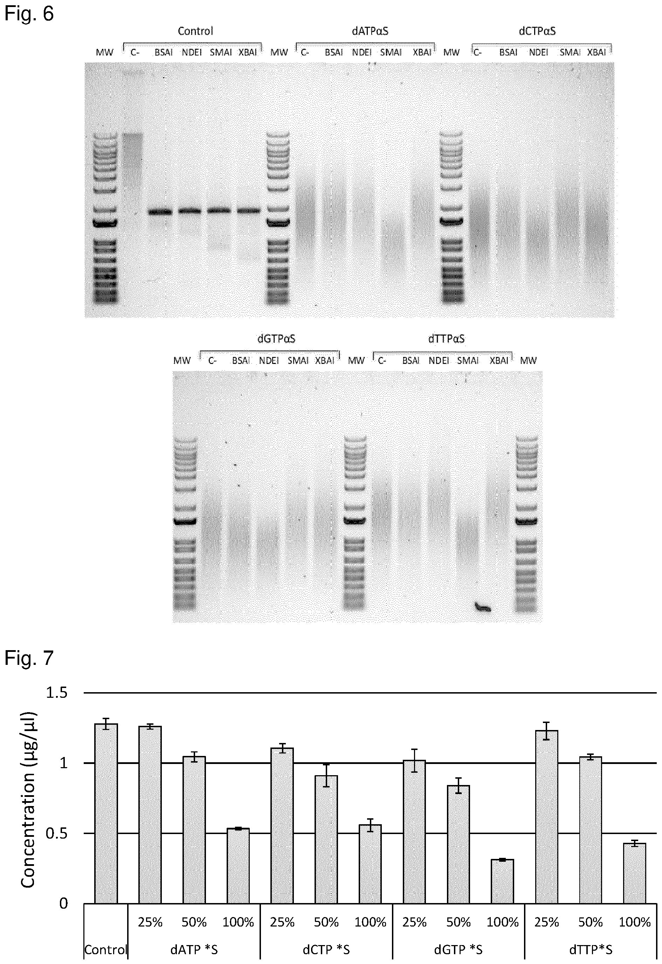

Figure 6 illustrates the digestion analysis of the amplified DNAs obtained with random primers by agarose gel electrophoresis (0.8%). Amplified DNAs were digested with several restriction enzymes (i.e. BsaI, NdeI, SmaI and XbaI) that only had one restriction site in the expression cassette. Therefore, a single band should have been seen in the gel reflecting the length of the cassette (2 kb approx.). Digestion conditions: reaction volume 25 µl, 2.5 µl reaction buffer CutSmart 10x (NEB), 400 ng DNA, 20 units BsaI-HFv2 (NEB), 20 units NdeI (NEB), 20 units SmaI (NEB), 20 units XbaI (NEB), incubation time and temperature: 60 min (BsaI) or 15 min (NdeI, SmaI and XbaI) at 25°C (SmaI) or 37°C (BsaI, NdeI and XbaI).
Figure 7 illustrates amplification yields in the presence of different proportions of unmodified and phosphorothioated nucleotides. Specifically, this Figure illustrates Picogreen quantification of the amplification yields obtained from Cre-derived circular DNA molecules using different proportions of unmodified 2'-deoxynucleotides-5'-triphosphate (dNTP) and protected 2'-deoxynucleotides-5'-(α-thio)-triphosphate (dNTP*S) for each of the four nucleotides. Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C.

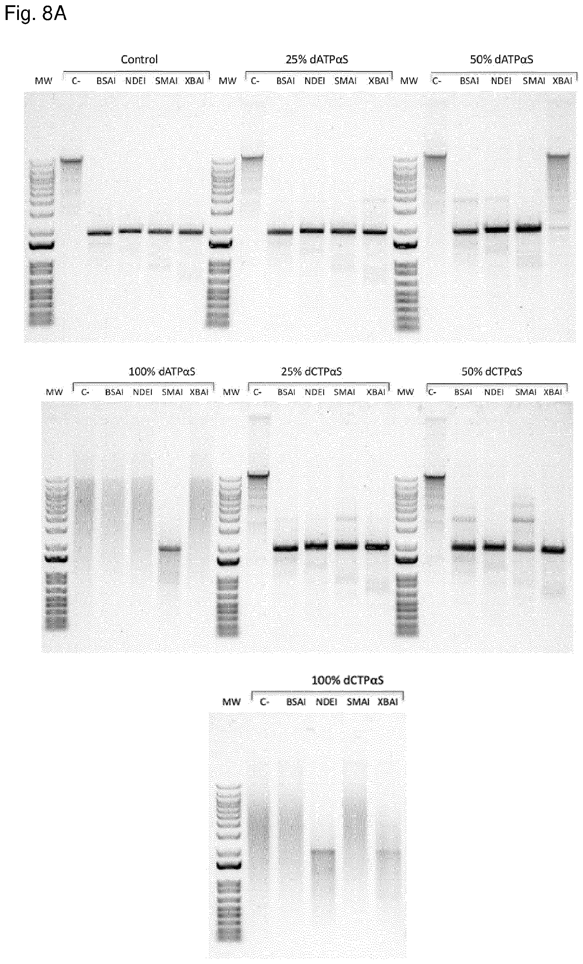

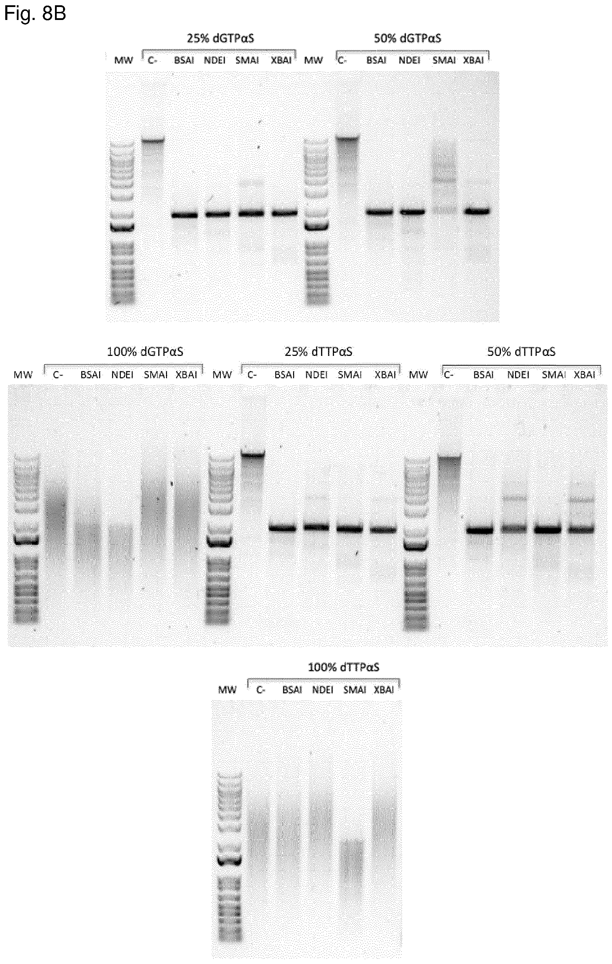

Figure 8 (Figures 8A and 8B) illustrates the digestion analysis of the amplified DNAs obtained with *Tth*PrimPol and different proportions of natural and protected nucleotides by agarose gel electrophoresis (0.8%). Amplified DNAs were digested with several restriction enzymes (i.e. BsaI, NdeI, SmaI and XbaI) that only had one restriction site in the expression cassette. Therefore, a single band should have been seen in the gel reflecting the length of the cassette (2 kb approx.). Digestion conditions: reaction volume 25 µl, 2.5 µl reaction buffer CutSmart 10x (NEB), 400 ng DNA, 20 units BsaI-HFv2 (NEB), 20 units NdeI (NEB), 20 units SmaI (NEB), 20 units XbaI (NEB), incubation time and temperature: 60 min (BsaI) or 15 min (NdeI, SmaI and XbaI) at 25°C (SmaI) or 37°C (BsaI, NdeI and XbaI).

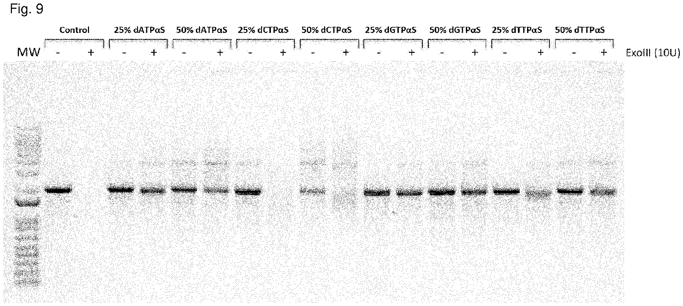

Figure 9 illustrates the protection analysis against the action of *E. coli* exonuclease III from amplified and digested DNAs having different proportions of unmodified and protected nucleotides. First, amplified DNAs were digested with BsaI. Digestion conditions: reaction volume 50 µl, 5 µl reaction buffer CutSmart 10x (NEB), 2 µg DNA, 40 units BsaI-HFv2 (NEB), incubation time and temperature: 30 min at 37°C. Then, 480 ng of Bsal-digested DNAs were further incubated with exonuclease III (10 units, NEB) to promote DNA degradation, as it was observed in the unprotected DNA control. Exonuclease incubation time and temperature: 30 min at 37°C and 20 min at 80°C.

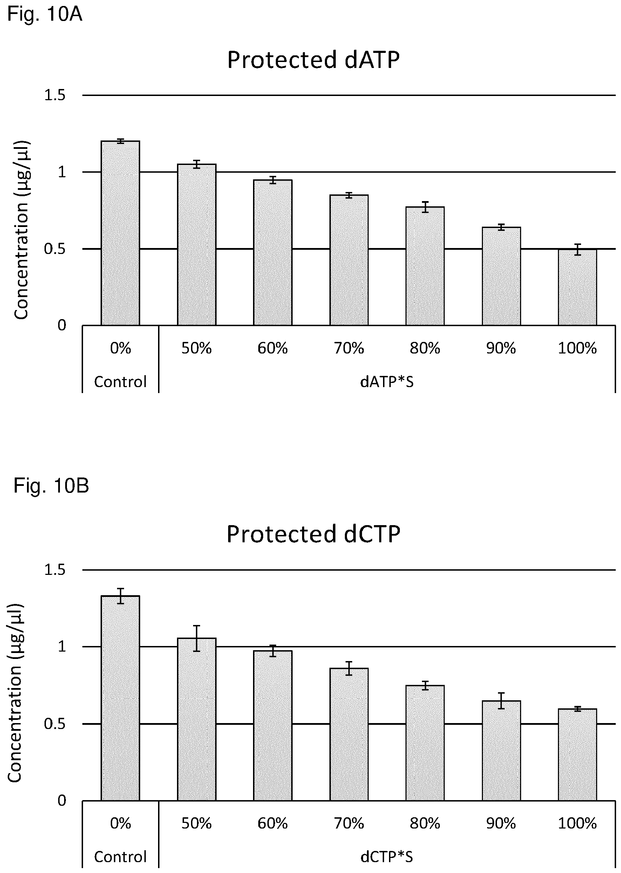

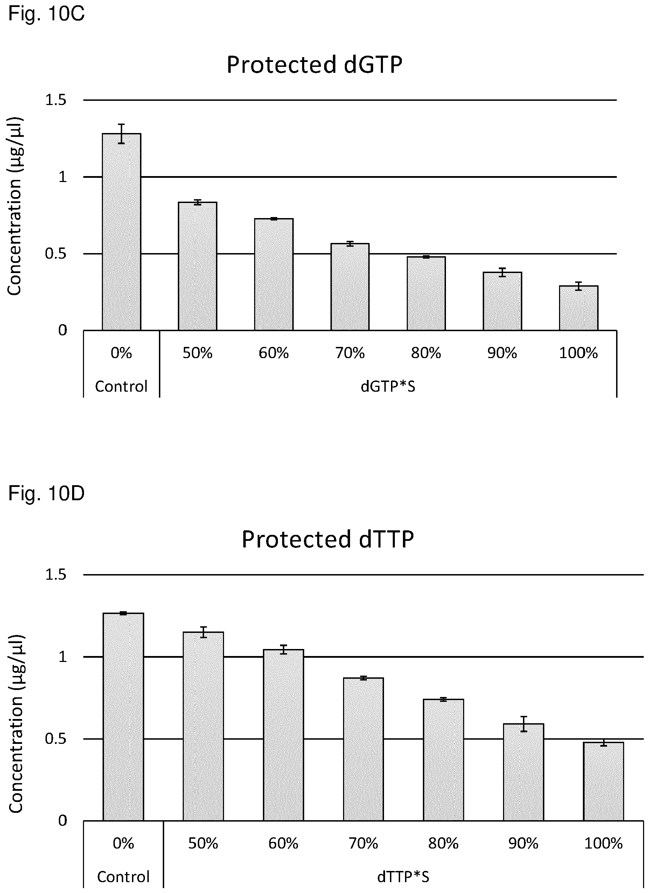

Figure 10 (Figures 10A-D) illustrates amplification yields in the presence of high levels of phosphorothioated nucleotides. Specifically, this Figure illustrates Picogreen quantification of the amplification yields obtained from Cre-derived circular DNA molecules using dNTP*S concentrations ranging from 50% to 100% for each of the four nucleotides. Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C.

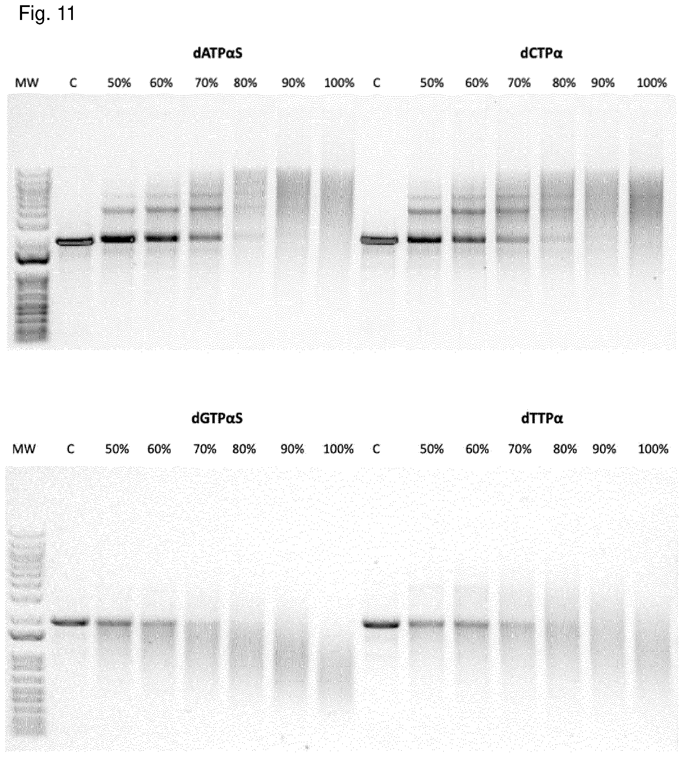

Figure 11 illustrates the digestion analysis by agarose gel electrophoresis of amplified DNAs obtained with phosphorothioated nucleotides (dNTP*S) concentrations ranging from 50% to 100%. Digestion conditions: reaction volume 50 µl, 5 µl reaction buffer CutSmart 10x (NEB), 2 µg DNA, 40 units Bsal-HFv2 (NEB), incubation time and temperature: 30 min at 37°C.

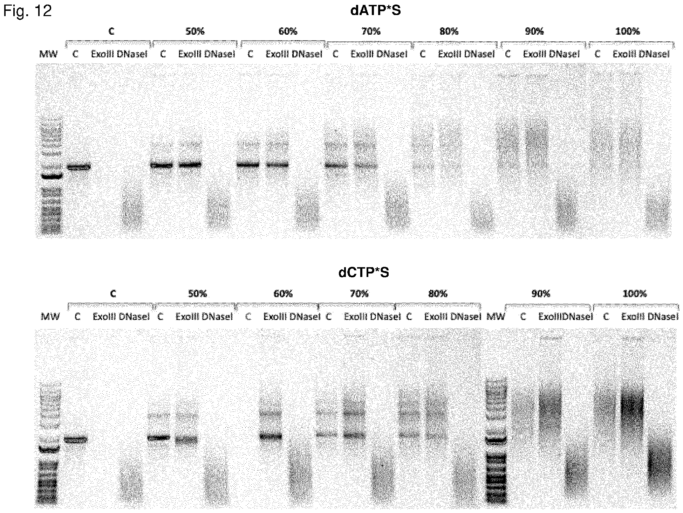

Figure 12 illustrates the protection analysis against the action of *E. coli* exonuclease III and bovine DNAse I from amplified and digested DNAs obtained with phosphorothioated nucleotides (dATP*S and dCTP*S) concentrations ranging from 50% to 100%. First, amplified DNAs were digested with Bsal. Digestion conditions: reaction volume 50 µl, 5 µl reaction buffer CutSmart 10x (NEB), 2 µg DNA, 40 units BsaI-HFv2 (NEB), incubation time and temperature: 30 min at 37°C. Then, 480 ng of BsaI-digested DNAs were further incubated with exonuclease III (10 units, NEB) or bovine DNAse I (0.0025 units, Ambion) supplemented with 5 mM CaCl₂. Incubation time and temperature: 30 min at 37°C.

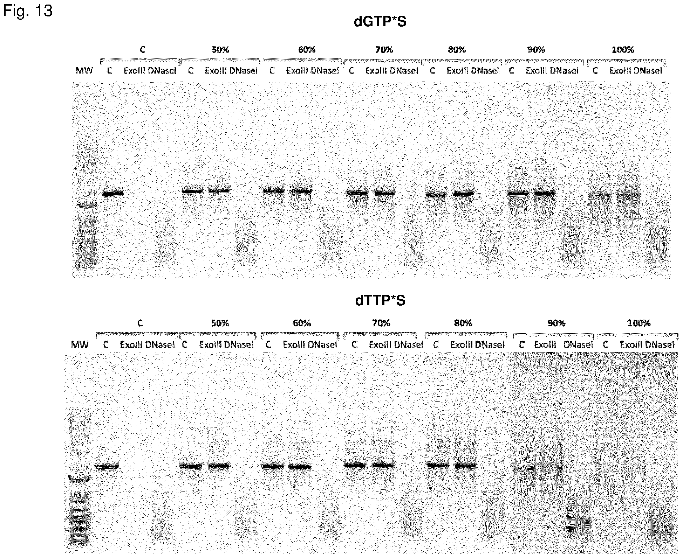

Figure 13 illustrates the protection analysis against the action of *E. coli* exonuclease III and bovine DNAse I from amplified and digested DNAs obtained with phosphorothioated nucleotides (dGTP*S and dTTP*S) concentrations ranging from 50% to 100%. First, amplified DNAs were digested with Bsal. Digestion conditions: reaction volume 50 µl, 5 µl reaction buffer CutSmart 10x (NEB), 2 µg DNA, 40 units BsaI-HFv2 (NEB), incubation time and temperature: 30 min at 37°C. Then, 480 ng of BsaI-digested DNAs were further incubated with exonuclease III (10 units, NEB) or bovine DNAse I (0.0025 units, Ambion) supplemented with 5 mM CaCl₂. Incubation time and temperature: 30 min at 37°C.

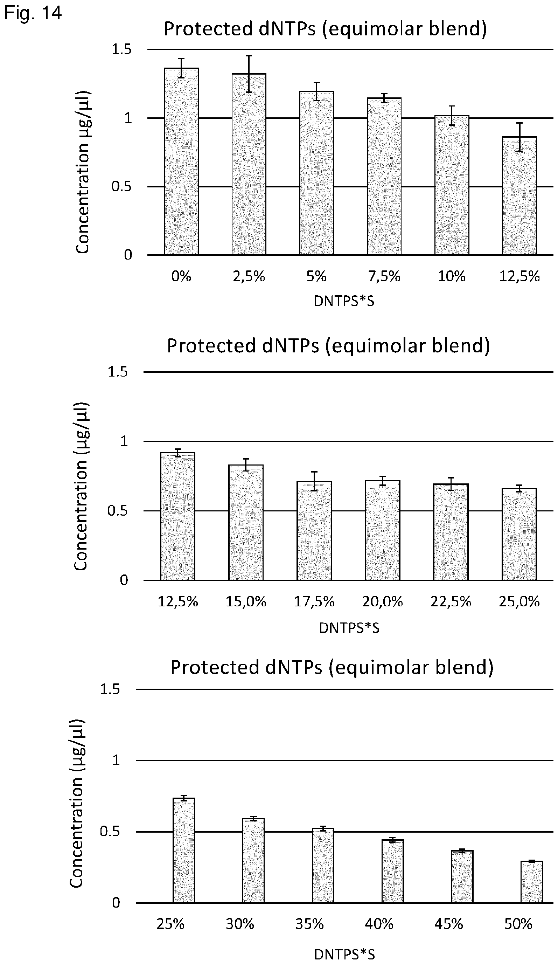

Figure 14 illustrates amplification yields in the presence of equimolar levels of phosphorothioated nucleotides. Specifically, this Figure illustrates Picogreen quantification of the amplification yields obtained from Cre-derived circular DNA molecules using equimolar blends of the four dNTP*S with concentrations ranging from 2.5% to 50%. Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C.

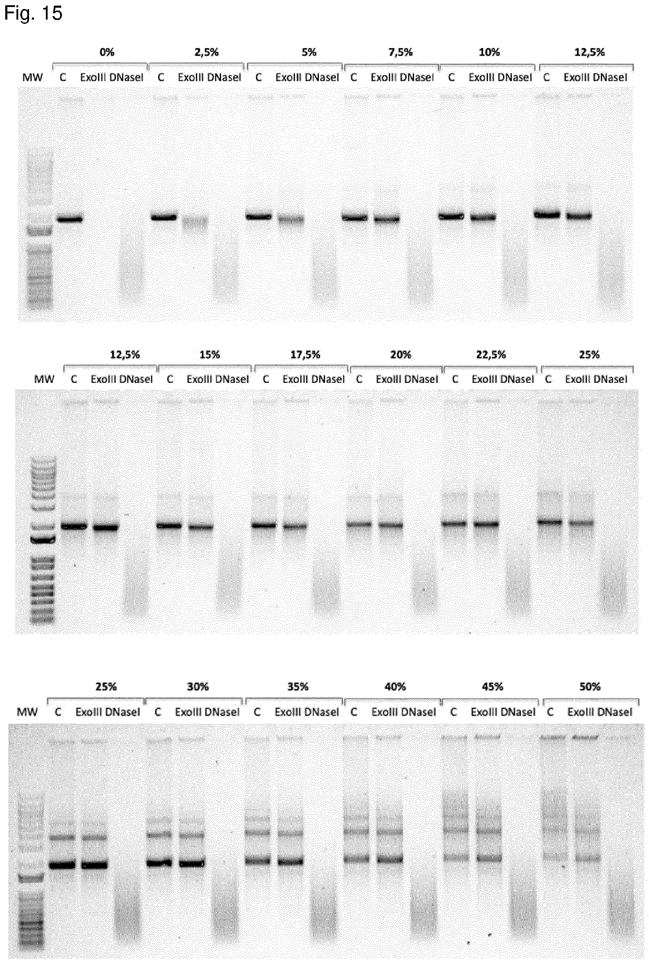

Figure 15 illustrates the protection analysis against the action of *E. coli* exonuclease III and bovine DNAse I from amplified and digested DNAs obtained with equimolar blends of the four dNTP*S with concentrations ranging from 2.5% to 50%. First, amplified DNAs were digested with BsaI. Digestion conditions: reaction volume 50 µl, 5 µl reaction buffer CutSmart 10x (NEB), 2 µg DNA, 40 units BsaI-HFv2 (NEB), incubation time and temperature: 30 min at 37°C. Then, 480 ng of Bsal-digested DNAs were further incubated with exonuclease III (10 units, NEB) or bovine DNAse I (0.0025 units, Ambion) supplemented with 5 mM CaCl₂. Incubation time and temperature: 30 min at 37°C.

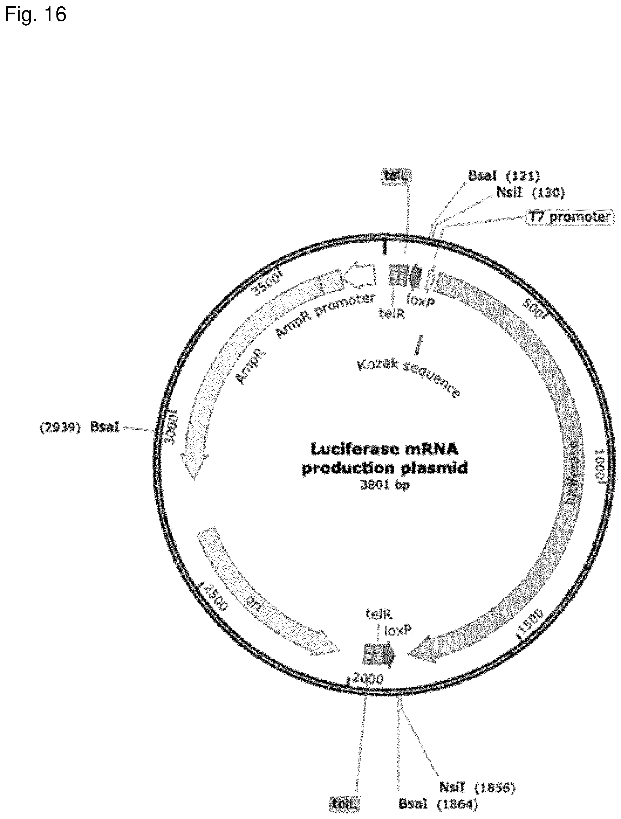

Figure 16 illustrates an exemplary template DNA molecule used in the methods described herein. The elements composing the plasmid DNA used as substrate to assess that DNA-containing protected 2'-deoxynucleotides-5'-(α-thio)-triphosphate can be transcribed in vitro. The expression cassette is formed by T7 promoter (SEQ ID NO: 9) and luciferase reporter gene (SEQ ID NO: 10). The expression cassette is flanked by Nsil restriction sites.

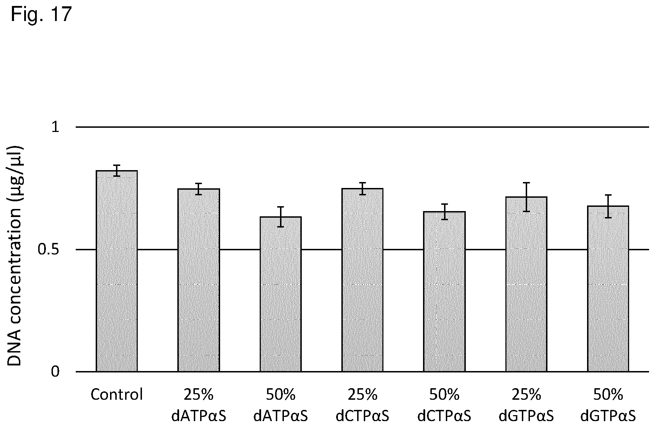

Figure 17 illustrates amplification yields in the presence of different proportions of phosphorothioated nucleotides. Specifically, this Figure illustrates Picogreen quantification of the amplification yields obtained from Cre-derived circular DNA molecules using different proportions of unmodified 2'-deoxynucleotides-5'-triphosphate (dNTP) and protected 2'-deoxynucleotides-5'-(α-thio)-triphosphate (dNTP*S) for dATP, dCTP and dGTP. Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C.

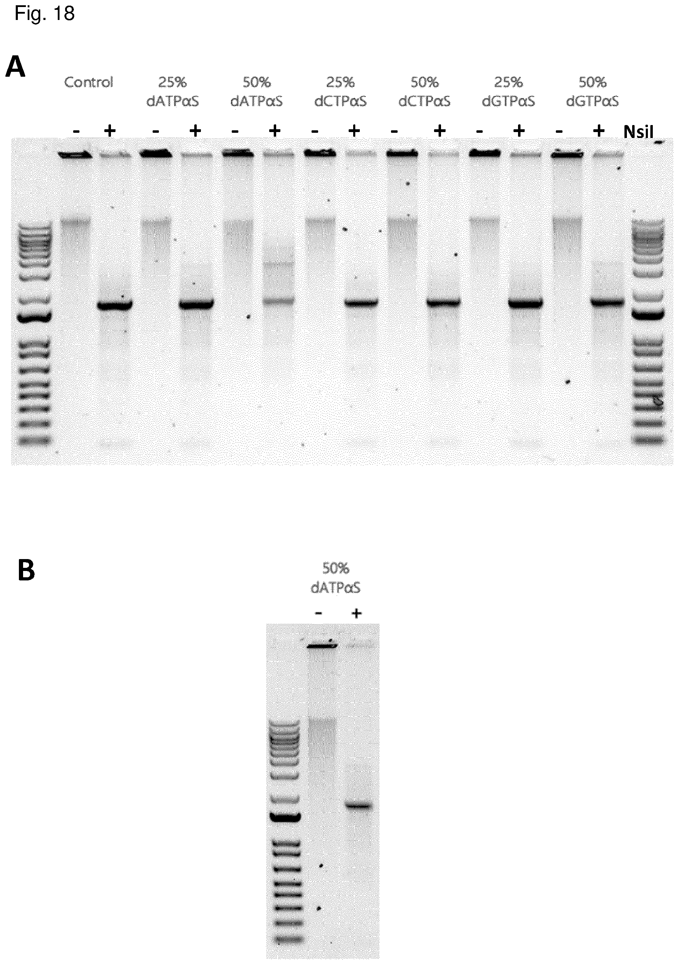

Figure 18 illustrates the digestion analysis of the amplified DNAs by agarose gel electrophoresis (0.8%). Amplified DNAs were digested with Nsil that had two restriction sites flanking the expression cassette. A single band corresponding to the fragment of interest (1.7 kb) was observed in all cases (part A), apart from 50% dATP*S, which required a second digestion (part B). Digestion conditions: reaction volume 500 µl, 50 µl reaction buffer CutSmart 10x (NEB), 50 µg DNA, 312 units Nsil (NEB), incubation time and temperature: 60 min at 37°C and 20 min at 80°C.

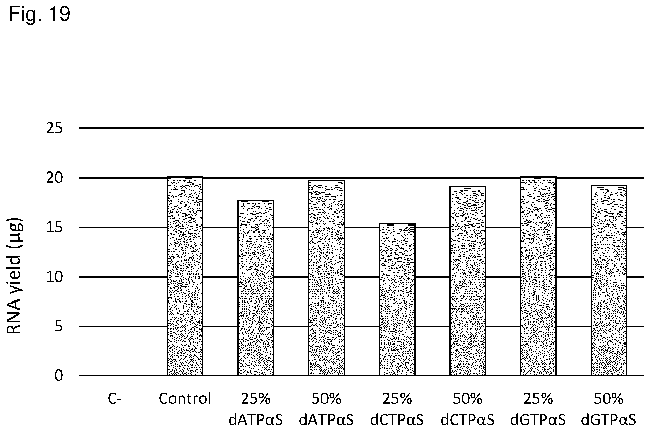

Figure 19 illustrates Qubit quantification of the mRNA generated during in vitro transcription using T7 RNA polymerase and the DNA products generated (see FIG. 17 and 18). Reaction conditions: reaction volume 20 µl, 2 µl reaction buffer T7 RNA pol reaction buffer 10x (NEB), 1 µg DNA, 100 T7 RNA polymerase (NEB), 2 mM NTPs, incubation time and temperature: 120 min at 37°C. Then, template DNA was removed by the addition of DNAse I (4 U, Ambion) and incubation at 37°C for 15 min.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

The invention is further disclosed in the following clauses:

### Products comprising protected nucleotides

1. A linear double-stranded DNA product comprising a sense strand and an antisense strand, wherein the linear double-stranded DNA product comprises one or more phosphorothioated nucleotides at internal positions in each strand.
2. The linear double-stranded DNA product of clause 1 further comprising a single cassette.
3. The linear double-stranded DNA product of clause 1 or 2, wherein the single cassette comprises a coding sequence.
4. The linear double-stranded DNA product of any one of clauses 1-3, wherein the cassette comprises a promoter and a coding sequence.
5. The linear double-stranded DNA product of any one of clauses 1-4, wherein the cassette comprises a promoter, a coding sequence, a ribosomal binding site and a translational termination sequence.
6. The linear double-stranded DNA product of any one of clauses 1-5, wherein the cassette is a mammalian expression cassette.
7. The linear double-stranded DNA product of any one of clauses 1-6, wherein the cassette comprises a single copy of the gene of interest.
8. The linear double-stranded DNA product of any one of clauses 1-7, wherein the cassette comprises a single copy of the gene of interest.
9. The linear double-stranded DNA product of any one of clauses 2-8, wherein the one or more phosphorothioated nucleotides at the internal positions in each strand may be selected from:
   (a) a 5'-end nucleotide of the cassette;
   (b) a 3'-end nucleotide of the cassette; and
   (c) a nucleotide outside of the cassette.
10. The linear double-stranded DNA product of any one of clauses 2-9, comprising at least two phosphorothioated nucleotides at internal positions in each strand, wherein the at least two phosphorothioated nucleotides at internal positions in each strand are selected from:
   (a) a 5'-end nucleotide of the cassette;
   (b) a 3'-end nucleotide of the cassette; and
   (c) one or more nucleotides outside of the cassette.
11. The linear double-stranded DNA product of clause 10, wherein, in the sense strand:
   (a) one of the at least two phosphorothioated nucleotides is the 5'-end nucleotide of the cassette; and/or
   (b) one of the at least two phosphorothioated nucleotides is in a first region of the sense strand, wherein the first region of the sense strand is 5' of the cassette.
12. The linear double-stranded DNA product of clause 10 or clause 11, wherein, in the sense strand:
   (a) one of the at least two phosphorothioated nucleotides is the 3'-end nucleotide of the cassette; and/or
   (b) one of the at least two phosphorothioated nucleotides is in a second region of the sense strand, wherein the second region of the sense strand is 3' of the cassette.
13. The linear double-stranded DNA product of clause 10, wherein:
   (a) in the sense strand one of the at least two phosphorothioated nucleotides is the 5'-end nucleotide of the cassette, and/or one of the at least two phosphorothioated nucleotides is in a first region of the sense strand, wherein the first region of the sense strand is 5' of the cassette;
   (b) in the sense strand one of the at least two phosphorothioated nucleotides is the 3'-end nucleotide of the cassette, and/or one of the at least two phosphorothioated nucleotides is in a second region of the sense strand, wherein the second region of the sense strand is 3' of the cassette;
   (c) in the antisense strand one of the at least two phosphorothioated nucleotides is the 5'-end nucleotide of the cassette, and/or one of the at least two phosphorothioated nucleotides is in a first region of the antisense strand, wherein the first region of the antisense strand is 5' of the cassette; and
   (d) in the antisense strand one of the at least two phosphorothioated nucleotides is the 3'-end nucleotide of the cassette, and/or one of the at least two phosphorothioated nucleotides is in a second region of the antisense strand, wherein the second region of the antisense strand is 3' of the cassette.
14. The linear double-stranded DNA product of any one of clauses 1-13, wherein the linear double-stranded DNA product comprises an overhang, optionally wherein the linear double-stranded DNA product comprises:
   (a) a 5' overhang and a blunt end;
   (b) two 5' overhangs;
   (c) a 3' overhang and a blunt end;
   (d) two 3' overhangs; and/or
   (e) a 5' overhang and a 3' overhang.
15. A complex molecule comprising the linear double-stranded DNA product of any one of clauses 1-14 and a functional portion, optionally wherein the functional portion is a binding molecule or a probe.
16. A nanoparticle comprising the linear double-stranded DNA product of any one of clauses 1-14, or the complex molecule of clause 15.
17. A library comprising at least two linear double-stranded DNA products, wherein each double-stranded DNA product is as defined in any one of clauses 1-14.
18. A library comprising at least two complex molecules, wherein each complex molecule is as defined in clause 15.
19. A composition comprising the linear double-stranded DNA product of any one of clauses 1-14, the complex molecule of clause 15, the nanoparticle of clause 16 or the library of clause 17 or 18.
20. A pharmaceutical composition comprising the composition of clause 19 and a pharmaceutically acceptable diluent.
21. A cell comprising the linear double-stranded DNA product of any one of clauses 1-14, the complex molecule of clause 15, the nanoparticle of clause 16, the library of clause 17 or 18, the composition of clause 19, or the pharmaceutical composition of clause 20.

### Uses of products comprising protected nucleotides

22. The linear double-stranded DNA product of any one of clauses 1-14, the complex molecule of clause 15, the nanoparticle of clause 16, the library of clause 17 or 18, the composition of clause 19, or the pharmaceutical composition of clause 20 for use in therapy.
23. The use of the linear double-stranded DNA product of any one of clauses 1-14, the complex molecule of clause 15, the nanoparticle of clause 16, the library of clause 17 or 18, the composition of clause 19, or the pharmaceutical composition of clause 20 in the production of RNA.
24. The use of clause 22, wherein the RNA is mRNA, miRNA, dsRNA, shRNA, gRNA or antisense RNA.
25. The use of the linear double-stranded DNA product of any one of clauses 1-14, the complex molecule of clause 15, the library of clause 17 or 18, the composition of clause 19, or the pharmaceutical composition of clause 20 in the production of a nanoparticle.
26. The use of the linear double-stranded DNA product of any one of clauses 1-14, the complex molecule of clause 15, the nanoparticle of clause 16, the library of clause 17 or 18, the composition of clause 19, or the pharmaceutical composition of clause 20 in the production of a vaccine.
27. The use of the linear double-stranded DNA product of any one of clauses 1-14, the complex molecule of clause 15, the nanoparticle of clause 16, the library of clause 17 or 18, the composition of clause 19, or the pharmaceutical composition of clause 20 in the production of a CAR-T cell.
28. The use of the linear double-stranded DNA product of any one of clauses 1-14, the complex molecule of clause 15, the nanoparticle of clause 16, the library of clause 17 or 18, the composition of clause 19, or the pharmaceutical composition of clause 20 in the production of a non-viral delivery system.
29. The use of the linear double-stranded DNA product of any one of clauses 1-14, the complex molecule of clause 15, the nanoparticle of clause 16, the library of clause 17 or 18, the composition of clause 19, or the pharmaceutical composition of clause 20 in the production of a viral delivery system.
30. The use of clause 28, wherein the viral delivery system is a viral vector, optional an AVV vector or a lentivirus vector.
31. The use of the linear double-stranded DNA product of any one of clauses 1-14, the complex molecule of clause 15, the nanoparticle of clause 16, the library of clause 17 or 18, the composition of clause 19, or the pharmaceutical composition of clause 20 in the production of a non-viral delivery system.
32. The use of the linear double-stranded DNA product of any one of clauses 1-14, the complex molecule of clause 15, the nanoparticle of clause 16, the library of clause 17 or 18, the composition of clause 19, or the pharmaceutical composition of clause 20 in the production of CRISPR delivery system.
33. The linear double-stranded DNA product of any one of clauses 1-14, the complex molecule of clause 15, the nanoparticle of clause 16, the library of clause 17 or 18, the composition of clause 19, or the pharmaceutical composition of clause 20 for use in cell therapy.
34. The linear double-stranded DNA product of any one of clauses 1-14, the complex molecule of clause 15, the nanoparticle of clause 16, the library of clause 17 or 18, the composition of clause 19, or the pharmaceutical composition of clause 20 for use in gene therapy.

### Methods of using the linear double-stranded DNA product

35. A method for in vitro transcription, comprising:
   (a) contacting the linear double-stranded DNA product of any one of clauses 1-14 with a polymerase; and
   (b) producing a transcription product.
36. A method for in vitro transcription, comprising:
   (a) contacting the complex molecule of clause 15 with a polymerase; and
   (b) producing a transcription product.
37. A method for protein expression, the method comprising:
   introducing the linear double-stranded DNA product of any one of clauses 1-14 into a prokaryotic cell or a eukaryotic cell or a cell-free protein expression system to generate a desired RNA or protein.
38. A method for protein expression, the method comprising:
   introducing the complex molecule of clause 15 into a prokaryotic cell or a eukaryotic cell or a cell-free protein expression system to generate a desired RNA or protein.
39. A cell transfection method comprising contacting a cell to be transfected with The linear double-stranded DNA product of any one of clauses 1-14, the complex molecule of clause 15, the nanoparticle of clause 16, the library of clause 17 or 18, the composition of clause 19, or the pharmaceutical composition of clause 20, and wherein the linear double-stranded DNA product, the complex molecule, the nanoparticle, the library, the composition, or the pharmaceutical composition is transfected into the cytosol of the cell.

### Methods of producing a linear double-stranded DNA product

40. A method for production of the linear double-stranded DNA product of any one of clauses 1-14, comprising:
   (a) rolling circle amplification of a DNA template molecule comprising at least one endonuclease target sequence in the presence of at least one type of a phosphorothioated nucleotide to generate a double-stranded DNA product,
   (b) contacting the double-stranded DNA product with at least one endonuclease to generate the linear double-stranded DNA product of any one of clauses 1-14, wherein the linear double-stranded DNA product has increased resistance to exonucleases.
41. A method for producing a linear double-stranded DNA product with protected ends, comprising the steps of:
   (a) addition of at least one homopolymeric sequence to a DNA template molecule
   (b) amplification of a DNA template molecule comprising at least one endonuclease target sequence and at the least one homopolymeric sequence in the presence of at least one type of a protected nucleotide to generate a double-stranded DNA product,
   (c) contacting the double-stranded DNA product with at least one endonuclease to generate the linear double-stranded DNA product, and
   (d) contacting the linear double-stranded DNA product with exonuclease to generate a linear double-stranded DNA product with protected ends.

### EXAMPLES

The following non-limiting examples further illustrate the present invention.

### Example 1: Phi29 DNA polymerase uses 2'-deoxynucleotides-5'-(α-thio)-triphosphate efficiently

Shown in FIG. 1 is the extension of a template/primer molecule by both wild-type and chimeric Phi29 DNA polymerases (Phi29DNApol) in the presence of different combinations of 2'-deoxynucleotides-5'-triphosphate (dNTP) and nucleotides in which an oxygen from the alpha phosphate has been replaced by a sulphur atom (2'-deoxynucleotides-5'-(α-thio)-triphosphate: dNTP*S). This change prevents DNA degradation by enzymes having 3'->5' or 5'->3' nucleolytic activity, since the phosphodiester bond formed is no longer cleavable by the enzyme when a dNTP*S is incorporated to a DNA primer.

Phi29 DNA polymerase chimera ("QualiPhi"; de Vega et al. "Improvement of ϕ29 DNA polymerase amplification performance by fusion of DNA binding motifs." Proceedings of the National Academy of Sciences 107.38 (2010): 16506-16511) was always able to extend a template/primer molecule by polymerizing up to 12 deoxynucleotides in the presence of different combinations of dNTP and dNTP*S. Wild-type Phi29 DNA polymerase was also able to show full extension of the DNA substrate for example, when 2'-deoxyguanosine-5'-(α-thio)-triphosphate (dGTP*S) was used. Phi29 DNA polymerase chimera was more effective in extending template/primer molecule than the corresponding wild-type DNA polymerase.

Reaction conditions: 2.5 nM DNA, 30 nM Phi29DNApol, 10 mM MgCl₂, 50 nM dNTPs, 5 min at 30°C. The reaction was run on 20% polyacrylamide gel in the presence of 8M urea.

### Example 2: Rolling circle amplification of template DNA molecules in the presence of different of 2'-deoxynucleotides-5'-(α-thio)-triphosphate can be triggered by TthPrimPol or random synthetic primers

Rolling circle amplification can be initiated by random synthetic primers (Dean et al. "Rapid amplification of plasmid and phage DNA using phi29 DNA polymerase and multiply-primed rolling circle amplification." Genome research 11.6 (2001): 1095-1099) or a DNA primase like *Tth*PrimPol (Picher et al. "TruePrime is a novel method for whole-genome amplification from single cells based on Tth PrimPol." Nature communications 7.1 (2016): 1-16) that synthesizes the primers for Phi29 DNA polymerase during the amplification reaction.

Shown in FIG. 4 are the amplification yields obtained from Cre-derived circular DNA molecules (template DNA molecules) substituting unprotected nucleotides for dNTP*S. The complete substitution of one dNTP for its protected counterpart caused a significant decrease in the amplification yield both using *Tth*PrimPol or random primers. This effect was further enhanced when two complementary dNTPs were replaced by the phosphorothioated versions, especially in the case of dATP and dTTP. When the four dNTP*S were used simultaneously, no amplification was observed in any of the cases. Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP or dNTP*S, incubation time and temperature: 20 hours at 30°C and 10 min at 65°C.

Shown in FIG. 5 are the amplification yields obtained in the presence of non-complementary dNTP*S pairs using *Tth*PrimPol or random primers. In the case of random primers, the amplification yields observed were similar with all the combinations tested. On the other hand, *Tth*PrimPol reactions were completely inhibited when dTTP*S was present in the pair (see FIG. 4 and 5). The other pairs tested without dTTP*S showed similar amplification levels (see FIG. 4 and 5). Amplification conditions: reaction volume 50 µl, denatured DNA sample (7.5 µl), 100 nM *Tth*PrimPol or 50 µM random synthetic hexamers, 200 nM QualiPhi Phi29DNApol, 2.5 units PPase (Thermo), 1 mM dNTP or dNTP*S, incubation time and temperature: 20 hours at 30°C and 10 min at 65°C.

Amplified DNAs were digested with several restriction enzymes (i.e. BsaI, NdeI, SmaI and XbaI) that only had one restriction site in the expression cassette. Therefore, a single band should have been seen in the gel reflecting the length of the cassette (2 kb approx.). Digestion conditions: reaction volume 25 µl, 2.5 µl reaction buffer CutSmart 10x (NEB), 400 ng DNA, 20 units Bsal-HFv2 (NEB), 20 units NdeI (NEB), 20 units SmaI (NEB), 20 units XbaI (NEB), incubation time and temperature: 60 min (BsaI) or 15 min (NdeI, SmaI and XbaI) at 25°C (SmaI) or 37°C (BsaI, NdeI and XbaI). Shown in FIG. 6 is the agarose gel electrophoresis analysis of the amplified DNAs obtained with random primers and digested with several restriction enzymes to release one unit size of the expression cassette (see FIG. 3). The complete substitution of one dNTP for its protected counterpart produced a significant reduction in the length of the amplified DNA, impeding the release of a single DNA band after digestion with several enzymes having only one restriction site in the expression cassette. Control DNA obtained with unmodified dNTPs showed the expected result in all cases (single 2-kb DNA band).

### Example 3: Rolling circle amplification of template DNA molecules in the presence of different concentrations of 2'-deoxynucleotides-5'-(α-thio)-triphosphate enables the production of linear double-stranded DNA product after digestion with restriction endonucleases

Shown in FIG. 7 are the amplification yields obtained from Cre-derived circular DNA molecules using different proportions of unmodified and protected dNTPs for each of the four nucleotides. In the four cases, the higher the concentration of the protected nucleotide, the lower the amplification yield observed. 25% of dATP*S and 25% dTTP*S did not produce any decrease in comparison to the control carried out with unmodified dNTPs only, and 25% of dGTP*S and 25% dCTP*S produced only a small decrease in comparison to the control. Thus, substitution of 25% of nucleotides to phosphorothioated nucleotides does not hinder amplification yields. Amplification conditions: reaction volume 50 µl, denatured DNA sample (7.5 µl), 100 nM TthPrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C.

Amplified DNAs were digested with several restriction enzymes (i.e. BsaI, NdeI, SmaI and XbaI) that only had one restriction site in the expression cassette. Therefore, a single band should have been seen in the gel reflecting the length of the cassette (2 kb approx.). Digestion conditions: reaction volume 25 µl, 2.5 µl reaction buffer CutSmart 10x (NEB), 400 ng DNA, 20 units Bsal-HFv2 (NEB), 20 units NdeI (NEB), 20 units SmaI (NEB), 20 units XbaI (NEB), incubation time and temperature: 60 min (BsaI) or 15 min (NdeI, SmaI and XbaI) at 25°C (SmaI) or 37°C (BsaI, NdeI and XbaI). Shown in FIG. 8 is the agarose gel electrophoresis analysis of the amplified DNAs obtained with *Tth*PrimPol and different concentrations of dNTP*S after digestion with several restriction enzymes to release the expression cassette. As it was previously observed with random primers (see FIG. 6), the complete substitution of one dNTP for its protected counterpart produced a significant reduction in the length of the amplified DNA, blocking the efficient release of a single DNA band after digestion with several restriction enzymes. Conversely, the concentration reduction to 25% or 50% facilitated obtaining the DNA of interest in almost all cases. The inefficient digestion of some DNA samples by certain enzymes can be explained by the fact that those enzymes perform their nucleolytic action in positions where the protected dNTP may be incorporated, preventing the digestion to occur.

### Example 4: Rolling circle amplification of template DNA molecules in the presence of different concentrations of 2'-deoxynucleotides-5'-(α-thio)-triphosphate enables the protection against exonucleases

Shown in FIG. 9 is the agarose gel electrophoresis analysis of the amplified and digested DNAs obtained with *Tth*PrimPol and different concentrations of dNTP*S. Amplified DNAs were first digested with Bsal and then incubated with *E. coli* exonuclease III, which is a double-stranded DNA specific exonuclease. Digestion conditions: reaction volume 50 µl, 5 µl reaction buffer CutSmart 10x (NEB), 2 µg DNA, 40 units BsaI-HFv2 (NEB), incubation time and temperature: 30 min at 37°C. Then, 480 ng of BsaI-digested DNAs were further incubated with exonuclease III (10 units, NEB) to promote DNA degradation, as it was observed in the unprotected DNA control. Exonuclease incubation time and temperature: 30 min at 37°C and 20 min at 80°C.

The presence of 25% or 50% of dATP*S, dGTP*S or dTTP*S prevented the DNA degradation observed in the absence of dNTP*S (control DNA). dCTP*S produced some protection at 50%.

Shown in FIG. 10 are the amplification yields obtained from Cre-derived circular DNA molecules using dNTP*S concentrations ranging from 50% to 100%. Amplification conditions: reaction volume 50 µl, denatured DNA sample (7.5 µl), 100 nM TthPrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C. As it was already observed in FIG. 7, the amplification yields were inversely proportional to the amount of dNTP*S in the four cases.

Shown in FIG. 11 is the agarose gel electrophoresis analysis of the amplified and digested DNAs obtained with *Tth*PrimPol and dNTP*S concentrations ranging from 50% to 100%. Digestion conditions: reaction volume 50 µl, 5 µl reaction buffer CutSmart 10x (NEB), 2 µg DNA, 40 units BsaI-HFv2 (NEB), incubation time and temperature: 30 min at 37°C. dNTP*S concentrations of 50% and higher produced a significant reduction in the capacity to obtain the target molecule of interest. GTP*S and dTTP*S caused a reduction in the length of the amplified DNA, preventing the production of the target molecule. On the other hand, dATP*S and dCTP*S reduced the capacity of Bsal to release single units of the expression cassette, since the addition of those dNTP*S can affect the point in which the enzyme acts in the expression cassette sequence. Thus, dimers, trimers and four-mers of the target DNA molecule appeared in the agarose gel. Thus, concentrations of dNTP*S below 50% are preferred.

Shown in FIG. 12 and 13 are the protection results against degradation mediated by *E. coli* exonuclease III and bovine DNAse I from the DNAs obtained with dNTP*S concentrations ranging from 50% to 100%. Digestion conditions: reaction volume 50 µl, 5 µl reaction buffer CutSmart 10x (NEB), 2 µg DNA, 40 units Bsal-HFv2 (NEB), incubation time and temperature: 30 min at 37°C. Then, 480 ng of Bsal-digested DNAs were further incubated with exonuclease III (10 units, NEB) or bovine DNAse I (0.0025 units, Ambion) supplemented with 5 mM CaCl2. Incubation time and temperature: 30 min at 37°C. Single units as well as dimers, trimers and four-mers of the target molecule produced with dATP*S and dCTP*S are completely protected against the action exonuclease III (see FIG. 12). Single units observed with dGTP*S and dTTP*S are also completely protected against exonuclease III (see FIG. 13). However, none of the samples showed protection against DNAse I, showing the same degradation pattern independently of the dNTP*S tested and its concentration.

### Example 5: Rolling circle amplification of template DNA molecules in the presence of equimolar blends of the four 2'-deoxynucleotides-5'-(α-thio)-triphosphate also enables protection against exonucleases

Shown in FIG. 14 are the amplification yields obtained from Cre-derived circular DNA molecules using equimolar blends of the four dNTP*S with concentrations ranging from 2.5% to 50%. Amplification conditions: reaction volume 50 µl, denatured DNA sample (7.5 µl), 100 nM TthPrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C. As it was already observed with increasing concentrations of single dNTP*S, the amplification yields were inversely proportional to the total amount of the four dNTP*S used.

Shown in FIG. 15 are the protection results against degradation mediated by *E. coli* exonuclease III and bovine DNAse I from the DNAs obtained with equimolar blends of the four dNTP*S with concentrations ranging from 2.5% to 50%. Digestion conditions: reaction volume 50 µl, 5 µl reaction buffer CutSmart 10x (NEB), 2 µg DNA, 40 units BsaI-HFv2 (NEB), incubation time and temperature: 30 min at 37°C. Then, 480 ng of Bsal-digested DNAs were further incubated with exonuclease III (10 units, NEB) or bovine DNAse I (0.0025 units, Ambion) supplemented with 5 mM CaCl₂. Incubation time and temperature: 30 min at 37°C. Even the lowest concentration tested (2.5%) showed certain level of protection against the action of exonuclease III, while full protection was already achieved at 10% or 12.5%. Higher concentrations showed also full protection, but dimers, trimers and four-mers appeared and increased proportionally to the concentration of dNTP*S. As it was already observed with single dNTP*S, none of the samples showed protection against DNAse I, showing the same degradation pattern independently of the concentration of the blend.

### Example 6: Protected DNA is properly transcribed in vitro by T7 RNA polymerase

Shown in FIG. 17 are the amplification yields obtained from Cre-derived circular DNA molecules using different proportions of unmodified and phosphorothioated dNTPs (dATP, dCTP and dGTP). Amplification conditions: reaction volume 50 µl, denatured DNA sample (7.5 µl), 100 nM TthPrimPol or 50 µM random synthetic hexamers, 200 nM QualiPhi Phi29DNApol, 2.5 units PPase (Thermo), 1 mM dNTP/dNTP*S, incubation time and temperature: 20 hours at 30°C and 10 min at 65°C. Template DNA included an expression cassette formed by T7 promoter and luciferase reporter gene (see FIG. 16). As it was already observed in FIG. 7 with a different expression cassette, the higher the concentration of the protected nucleotide, the lower the amplification yield observed. The decrease in the amplification yield was similar for the three dNTPs tested at each concentration.

Shown in FIG. 18 is the agarose gel electrophoresis analysis of the amplified DNAs after digestion with Nsil to release the molecule suitable to perform transcription in vitro with T7 RNA polymerase. Digestion conditions: reaction volume 500 µl, 50 µl reaction buffer CutSmart 10x (NEB), 50 µg DNA, 312 units Nsil (NEB), incubation time and temperature: 60 min at 37°C and 20 min at 80°C. A single band corresponding to the fragment of interest (1.7 kb) was observed in all cases (part A), apart from 50% dATP*S, which required a second digestion under the same conditions (part B). Digested DNAs were purified and used as templates in the in vitro transcription reaction with T7 RNA polymerase.

Shown in FIG. 19

## Claims

1. A linear double-stranded DNA product comprising a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette; and
wherein the cassette comprises a coding sequence.

2. The linear double-stranded DNA product of claim 1, wherein in the sense strand:
(a) one of the at least two phosphorothioated nucleotides is the 5'-end nucleotide of the cassette; and/or
(b) one of the at least two phosphorothioated nucleotides is in a first region of the sense strand, wherein the first region of the sense strand is 5' of the cassette.

3. The linear double-stranded DNA product of claim 1 or claim 2, wherein in the sense strand:
(a) one of the at least two phosphorothioated nucleotides is the 3'-end nucleotide of the cassette; and/or
(b) one of the at least two phosphorothioated nucleotides is in a second region of the sense strand, wherein the second region of the sense strand is 3' of the cassette.

4. The linear double-stranded DNA product of claim 1, wherein:
(a) in the sense strand one of the at least two phosphorothioated nucleotides is the 5'-end nucleotide of the cassette, and/or one of the at least two phosphorothioated nucleotides is in a first region of the sense strand, wherein the first region of the sense strand is 5' of the cassette;
(b) in the sense strand one of the at least two phosphorothioated nucleotides is the 3'-end nucleotide of the cassette, and/or one of the at least two phosphorothioated nucleotides is in a second region of the sense strand, wherein the second region of the sense strand is 3' of the cassette;
(c) in the antisense strand one of the at least two phosphorothioated nucleotides is the 5'- end nucleotide of the cassette, and/or one of the at least two phosphorothioated nucleotides is in a first region of the antisense strand, wherein the first region of the antisense strand is 5' of the cassette; and
(d) in the antisense strand one of the at least two phosphorothioated nucleotides is the 3'- end nucleotide of the cassette, and/or one of the at least two phosphorothioated nucleotides is in a second region of the antisense strand, wherein the second region of the antisense strand is 3' of the cassette.

5. The linear double-stranded DNA product of any one of claims 1-4, wherein the linear double-stranded DNA product comprises an overhang, optionally wherein the linear double-stranded DNA product comprises:
(a) a 5' overhang and a blunt end;
(b) two 5' overhangs;
(c) a 3' overhang and a blunt end;
(d) two 3' overhangs; and/or
(e) a 5' overhang and a 3' overhang.

6. A complex molecule comprising the linear double-stranded DNA product of any one of claims 1-5 and a functional portion, optionally wherein the functional portion is a binding molecule or a probe.

7. A nanoparticle comprising the linear double-stranded DNA product of any one of claims 1-5, or the complex molecule of claim 6.

8. A library comprising at least two linear double-stranded DNA products, wherein each double-stranded DNA product is as defined in any one of claims 1-5.

9. The linear double-stranded DNA product of any one of claims 1-5, the complex molecule of claim 6, the nanoparticle of claim 7, or the library of claim 8 for use in therapy.

10. The use of the linear double-stranded DNA product of any one of claims 1-5, the complex molecule of claim 6, the nanoparticle of claim 7, or the library of claim 8 in the production of RNA, optionally wherein the RNA is mRNA.

11. The use of the linear double-stranded DNA product of any one of claims 1-5, the complex molecule of claim 6, the nanoparticle of claim 7, or the library of claim 8 in the production of a nanoparticle.

12. A method for in vitro transcription, comprising:
(a) contacting the linear double-stranded DNA product of any one of claims 1-5 with a polymerase; and
(b) producing a transcription product.

13. A method for protein expression, the method comprising:
introducing the linear double-stranded DNA product of any one of claims 1-5 into a prokaryotic cell or a eukaryotic cell or a cell-free protein expression system to generate a desired RNA or protein.

14. A method for production of the linear double-stranded DNA product of any one of claims 1-5, comprising:
(a) rolling circle amplification of a DNA template molecule comprising at least one endonuclease target sequence in the presence of at least one type of a phosphorothioated nucleotide to generate a double-stranded DNA product,
(b) contacting the double-stranded DNA product with at least one endonuclease to generate the linear double-stranded DNA product of any one of claims 1-5, wherein the linear double-stranded DNA product has increased resistance to exonucleases.
